# EUROPÄISCHE PATENTANMELDUNG

(11) **EP 4 749 637 A1**
(43) Veröffentlichungstag der Anmeldung: **27.05.2026**
(21) Anmeldenummer: 24215149.6
(22) Anmeldetag: 25.11.2024
(51) Int. Cl.: G16H 20/30, G16H 40/63, G16H 50/20, G16H 50/30

(54) **VERRINGERUNG VON RISIKEN MEDIZINISCHER EINGRIFFE**

(71) Anmelder: Capreolos GmbH, 60347 Frankfurt am Main (DE)
(72) Erfinder: Schnitzbauer, Andreas, 45134 Essen (DE)
(74) Vertreter: Eisenführ Speiser

(57) **Zusammenfassung**

Die Erfindung bezieht sich auf ein System, ein Verfahren und ein Computerprogramm zur Verringerung von Risiken medizinischer Eingriffe. Erfindungsgemäß werden Werte medizinischer Indikatoren (10, 11, 12) eines prähabilitativen Zustand eines Patienten bereitgestellt, wobei darauf basierend eine Risikoklasse bestimmt wird, die indikativ für ein Risiko ist, das ein geplanter medizinischer Eingriff für den Patienten birgt. Basierend auf den Werten der medizinischen Indikatoren und/oder der Risikoklasse (20) werden Parameter (30) prähabilitativer Maßnahmen für den Patienten bestimmt, wobei die Maßnahmen geeignet sind, das Risiko, das der geplante medizinische Eingriff für den Patienten birgt, zu senken.

## Beschreibung

### GEBIET DER ERFINDUNG

Die Erfindung bezieht sich auf ein System, ein Verfahren und ein Computerprogramm zur Verringerung von Risiken medizinischer Eingriffe. Weitere Aspekte der vorliegenden Offenbarung betreffen ein Endgerät zur Unterstützung eines Patienten bei der Durchführung prähabilitativer Maßnahmen und ein Endgerät zur Unterstützung eines Arztes bei der Beurteilung eines Risikos, das ein geplanter medizinischer Eingriff auf einen Patienten birgt.

### TECHNISCHER HINTERGRUND

Es ist bekannt, dass Patienten nach der Durchführung eines medizinischen Eingriffs an ihnen durch den gezielten Einsatz digitaler Anwendungen dabei unterstützt werden können, sich von dem medizinischen Eingriff zu erholen. Digitale Anwendungen können insbesondere die Durchführung von Maßnahmen durch den Patienten überwachen und anleiten, die geeignet sind, körperliche Einschränkungen, die sich aus dem medizinischen Eingriff, einschließlich eines möglicherweise vorangegangen Krankenhausaufenthalts, ergeben können, abzumildern und/oder möglichst schnell zu überwinden (Rehabilitationsmaßnahmen). Ein Risiko, dass durch einen medizinischen Eingriff zwar möglicherweise ein mit dem Eingriff verfolgtes medizinisches Hauptziel erreicht wird, der Patient aber dennoch körperliche Einschränkungen von dem medizinischen Eingriff davonträgt, d.h. insbesondere eine Dauer und Schwere der körperlichen Einschränkungen, kann also mit entsprechenden digitalen Anwendungen verringert werden.

### ZUSAMMENFASSUNG DER ERFINDUNG

Vor dem beschriebenen Hintergrund kann es als eine der vorliegenden Erfindung zugrundeliegende Aufgabe angesehen werden, Risiken medizinischer Eingriffe weiter zu verringern.

Gemäß einem ersten Aspekt der Erfindung ist ein System zur Verringerung von Risiken medizinischer Eingriffe vorgesehen, das eine Bereitstellungseinheit zum Bereitstellen von Werten einer Mehrzahl medizinischer Indikatoren aufweist, die indikativ für einen prähabilitativen Zustand eines Patienten sind, und zudem eine Klassifizierungseinheit zum Bestimmen einer Risikoklasse für den Patienten basierend auf den Werten der Mehrzahl medizinischer Indikatoren, wobei die Risikoklasse indikativ für ein Risiko ist, das ein geplanter medizinischer Eingriff für den Patienten birgt. Zusätzlich weist das System eine Parametrisierungseinheit zum Bestimmen von Werten einer Mehrzahl von Parametern prähabilitativer Maßnahmen für den Patienten basierend auf den Werten der Mehrzahl medizinischer Indikatoren und/oder der Risikoklasse auf, wobei die Maßnahmen geeignet sind, das Risiko, das der geplante medizinische Eingriff für den Patienten birgt, zu senken.

Die bestimmte Risikoklasse erleichtert es einem behandelnden Arzt, für eine Vielzahl von Patienten eine Entscheidungshilfe zu erhalten, ob diese zu einem jeweils geplanten medizinischen Eingriff zugelassen werden sollten. Dies verringert das Risiko, dass medizinische Eingriffe an Patienten vorgenommen werden, obwohl die Patienten noch in einem körperlich-gesundheitlichen Zustand sind, der dazu führen könnte, dass sie von dem medizinischen Eingriff Schaden davontragen. Die Bestimmung von Parametern prähabilitativer Maßnahmen für den jeweiligen Patienten unterstützt zusätzlich die Patienten dabei, ihren körperlich-gesundheitlichen Zustand zu verbessern und so das Risiko zu verringern, dass sie von dem medizinischen Eingriff Schaden davontragen. Das System erlaubt also in doppelter Hinsicht eine Verringerung des Risikos medizinischer Eingriffe für Patienten. Der Ansatz kann als komplementär zu den bekannten, erst nach Durchführung eines medizinischen Eingriffs durchgeführten Rehabilitationsmaßnahmen angesehen werden, und erlaubt insoweit eine weitere Verringerung des Risikos medizinischer Eingriffe für Patienten. Der Begriff der "prähabilitativen" Maßnahmen ist daher in Anlehnung an die bekannten, "rehabilitativen" Maßnahmen gewählt. Die prähabilitativen Maßnahmen könnten aber auch als Vorsorge- oder Vorbereitungsmaßnahmen bezeichnet werden.

Bevorzugt ist das System computerimplementiert, d.h. ein Datenverarbeitungssystem. Auf diese Weise erlaubt es eine effiziente Betreuung einer Vielzahl von Patienten. Das Risiko medizinischer Eingriffe, das mit dem System verringert werden kann, kann dann auch als ein kumulatives oder kollektives Risiko verstanden werden.

Die für den prähabilitativen Zustand des Patienten indikativen medizinischen Indikatoren können auch als Kennwerte des Patienten verstanden werden, die mit dem Risiko, das der geplante medizinische Eingriff für den Patienten birgt, korrelieren. Ob eine solche Korrelation vorliegt, kann für einen gegebenen Indikator ermittelt werden, beispielsweise indem jeweils ein Wert dieses Indikators für eine Vielzahl von Patienten vor für die Patienten geplanten medizinischen Eingriffen bestimmt wird und die bestimmten Werte mit unerwünschten Folgen der medizinischen Eingriffe für die Patienten ins Verhältnis gesetzt werden.

Vorzugsweise ist die Bereitstellungseinheit eingerichtet, den Wert eines oder mehrerer der folgenden medizinischen Indikatoren für den Patienten bereitzustellen: Geburtsjahr oder Alter, Größe, Gewicht, Body-Mass-Index, ob der Patient Raucher ist, Ruhepuls, ECOG-Status, TUG-Ergebnis, Hämoglobingehalt im Blut, ob der Patient herzfrequenzbeeinflussende, insbesondere herzfrequenzsenkende, Medikamente einnimmt, und RAI-Score. Es könnten auch verwandte Indikatoren, Kennwerte oder Angaben bereitgestellt werden, die lediglich indikativ für einen oder mehrere der genannten konkreten Indikatoren, oder aber noch spezifischer sind. Die genannten Indikatoren haben sich aber als besonders geeigneter Kompromiss zwischen einfacher Bereitstellbarkeit und genauer Charakterisierung des prähabilitativen Zustands von Patienten herausgestellt.

Wie der ECOG-Status für einen Patienten bestimmt wird, ist bekannt. Sein Wert gibt das allgemeine Wohlbefinden und die Aktivität eines Patienten im Alltag an. Die Werteskala und die zugehörigen Kriterien wurden von der Eastern Cooperative Oncology Group (ECOG) aufgestellt. Auch der TUG-Test ist bekannt, wobei TUG für "Timed up and go" steht. Der TUG-Test ist ein Test der Mobilität einer Person. Hinsichtlich der Definition des RAI-Scores wird beispielhaft auf den Artikel *"*Development and Initial Validation ofthe Risk Analysis Index for Measuring Frailty in Surgical Populations" von D. E. Hall et al. JAMA Surgery 2017; 152(2):175-182 verwiesen, der hiermit in Gänze in die vorliegende Offenbarung mit einbezogen sei. Der von der Bereitstellungseinheit bereitgestellte RAI-Score könnte also beispielsweise ein RAI-A oder ein RAI-C sein, wie er in diesem Artikel beschrieben ist. Grundsätzlich könnte aber jede Entwicklungsstufe des RAI-Scores verwendet werden. Die Bereitstellungseinheit könnte im Allgemeinen also den RAI-Score gemäß einer beliebigen seiner Entwicklungsstufen bereitstellen.

Vorzugsweise ist die Bereitstellungseinheit eingerichtet, einen oder mehrere der Werte der Mehrzahl der medizinischen Indikatoren basierend auf einer Nutzereingabe zu bestimmen. Die Nutzereingabe kann beispielsweise durch einen Arzt oder medizinisches Personal getätigt werden, etwa infolge entsprechender Messungen, einer Befragung des Patienten und der Durchführung des TUG-Tests. Zusätzlich oder alternativ zu Nutzereingaben können auch für die Bestimmung benötigte Daten aus einer Datenbank abgerufen werden. Auf Basis der Nutzereingaben und/oder der abgerufenen Daten kann dann, insoweit diese nicht bereits einem Wert der medizinischen Indikatoren entsprechen, eine automatische Bestimmung der Werte der medizinischen Indikatoren erfolgen. Zu diesem Zweck kann die Bereitstellungseinheit mit einer entsprechend eingerichteten Indikatorbestimmungseinheit kommunizieren oder selbst als solche fungieren. Insbesondere für die Bestimmung des verhältnismäßig komplexeren RAI-Scores können beispielsweise die hierfür gemäß dem oben genannten Artikel vorgesehenen Angaben eingegeben oder abgerufen werden, wobei auf dieser Basis eine automatische Bestimmung des RAI-Scores nach den in dem Artikel angegeben Regeln erfolgen kann.

Der RAI-Score ist ein Wert, der als für die Gebrechlichkeit eines Patienten indikativer Kennwert verstanden werden kann. Grundsätzlich ist neben dem RAI -Score auch die Verwendung anderer solcher Indikatoren denkbar. Jedenfalls kann bevorzugt sein, dass die Bereitstellungseinheit eingerichtet ist, als einen der Werte der Mehrzahl medizinischer Indikatoren einen für die Gebrechlichkeit des Patienten indikativen Kennwert bereitzustellen, und diesen basierend auf einer Nutzereingabe zu bestimmen, die indikativ für einen oder mehrere der folgenden Umstände ist: ob der Patient Krebs hat, ob der Patient in den zurückliegenden drei Monaten einen unbeabsichtigten Gewichtsverlust erlitten hat, ob bei dem Patienten ein Nierenversagen vorliegt, ob bei dem Patienten eine Herzinsuffizienz vorliegt, ob der Patient unter Appetitlosigkeit leidet, ob der Patient unter Kurzatmigkeit, insbesondere in Ruhe, leidet, ob der Patient unabhängig wohnt, das heißt, keine medizinische Alltagsversorgung in Anspruch nimmt, und falls nicht, ob der Patient a) in einer professionellen Pflegeeinrichtung, b) in betreutem Wohnen oder c) in einem Pflegeheim lebt, und ob der Patient hierfür innerhalb der zurückliegenden drei Monate zugelassen wurde, ob eine Pflegeabhängigkeit des Patienten im Alltag hinsichtlich Mobilität, Nahrungsaufnahme, Toilettenbenutzung und/oder persönliche Hygiene vorliegt, und ob der kognitive Zustand des Patienten sich innerhalb der zurückliegenden drei Monate verschlechtert hat. Diese Umstände sind auch Grundlage des RAI-Scores.

Die Klassifizierungseinheit ist eingerichtet, die Risikoklasse für den Patienten basierend auf den bereitgestellten Werten der Mehrzahl medizinischer Indikatoren zu bestimmen. Die Risikoklasse kann auch als Maß, oder weiterer Score, für das Risiko verstanden werden, das der geplante medizinische Eingriff für den Patienten birgt. Dadurch, dass die Risikoklasse basierend auf mehreren medizinischen Indikatoren bestimmt wird, erlaubt sie die Bündelung eines umfassenden Informationsstands über den prähabilitativen Zustand des Patienten. Vorzugsweise wird das Risiko in lediglich drei Klassen unterteilt. Dies erlaubt eine besonders leichte Orientierung für den Arzt. Beispielsweise kann anhand der Risikoklasse bestimmt werden, ob ein Patient ein eher niedriges oder ein eher hohes Risiko trägt. Mit anderen Worten kann basierend auf der Risikoklasse wiederum eine Eingruppierung von Patienten in Risikogruppen erfolgen, wobei bereits zwei Risikogruppen ausreichen können. In einer bevorzugten Ausführungsform werden als medizinische Indikatoren das TUG-Testergebnis, der ECOG-Score, der Hämoglobinwert und der RAI-Score bereitgestellt, und die Risikoklasse wird basierend auf diesen vier Indikatoren bestimmt. Beispielsweise können dazu das TUG-Testergebnis und der Hämoglobinwert ebenfalls in eine ganzzahlige, dimensionslose (und damit von der Wahl physikalischer Einheiten unabhängige) Skala überführt werden, um dann eine Summe über alle Indikatoren (d.h. die jeweiligen ganzen, einheitenlosen Zahlen) zu bilden, wobei die Summe als Risikoklasse angenommen wird. Basierend auf der in Form der Summe vorliegenden Risikoklasse und einem vorgegebenen Schwellwert kann der jeweilige Patient dann zusätzlich in eine Risikogruppe eingeteilt werden, beispielsweise "hoch" oder "niedrig".

Die Parametrisierungseinheit ist eingerichtet, Parameterwerte prähabilitativer Maßnahmen für den Patienten auf Basis der von der Bereitstellungseinheit bereitgestellten medizinischen Indikatoren und/oder der von der Klassifizierungseinheit bestimmten Risikoklasse zu bestimmen. Eine prähabilitative Maßnahme wird vorliegend als eine Maßnahme verstanden, die geeignet ist, das Risiko, das der geplante medizinische Eingriff für den Patienten birgt, zu senken. Ob dies der Fall ist, kann für eine gegebene Maßnahme ermittelt werden, beispielsweise indem die Maßnahme von einer Vielzahl von Patienten vor für die Patienten geplanten medizinischen Eingriffen durchgeführt wird und die Häufigkeit unerwünschter Folgen der medizinischen Eingriffe für diese Patienten ins Verhältnis zu einer entsprechenden Häufigkeit gesetzt wird, die ohne die Maßnahme zu erwarten gewesen wäre. Letztere kann anhand anderer Patienten bestimmt worden sein.

Vorzugsweise beziehen sich die prähabilitativen Maßnahmen auf ein körperliches Training des Patienten. Insbesondere kann das körperliche Training ein kardiovaskuläres Training sein. Die körperliche Leistungsfähigkeit eines Patienten und insbesondere seines kardiovaskulären Systems hat sich als wesentlicher Bestimmungsfaktor für Risiken herausgestellt, die medizinische Eingriffe für sie bergen. Patienten mit einem leistungsfähigen Herz-Kreislauf-System haben sich als weniger anfällig dafür herausgestellt, gesundheitliche Schäden aus medizinischen Eingriffen davonzutragen. Die Dauer körperlich-gesundheitlicher Einschränkungen nach medizinischen Eingriffen ist bei ihnen erwartungsgemäß verkürzt.

Bevorzugt ist die Parametrisierungseinheit eingerichtet, basierend auf den Werten der Mehrzahl medizinischer Indikatoren und/oder der Risikoklasse Herzfrequenz-Schwellwerte als Parameter für das kardiovaskuläre Training zu bestimmen. Die prähabilitativen Maßnahmen beziehen sich also vorzugsweise auf ein kardiovaskuläres Training, das von dem Patienten anhand von Herzfrequenz-Schwellwerten durchzuführen ist, wobei die Herzfrequenz-Schwellwerte basierend auf den Werten der medizinischen Indikatoren und/oder der Risikoklasse bestimmt werden. Auf diese Weise kann ein individuell abgestimmtes Training erfolgen, das für den Patienten dennoch leicht durchzuführen ist. Zur Durchführung des Trainings muss der Patient lediglich seine Herzfrequenz überwachen und mit den vorgegebenen Schwellwerten vergleichen, wobei der Vergleich durch technische Hilfsmitten wie beispielsweise eine auf einem Endgerät des Nutzers installierte Nutzeranwendung automatisiert vorgenommen werden kann. Die Nutzeranwendung kann die Herzfrequenz-Schwellwerte übergeben bekommen haben, beispielsweise von einem oder mehreren Servern, die in der Cloud implementiert sein können. Der wenigstens eine Server oder ein auf diesem ausgeführtes Programm könnte in einer solchen Ausführungsform auch als Backend bezeichnet werden, während das Endgerät des Nutzers oder die Nutzeranwendung, die auf diesem Endgerät ausgeführt wird, als Frontend bezeichnet werden könnte.

Die Parametrisierungseinheit kann insbesondere eingerichtet sein, die Herzfrequenz-Schwellwerte basierend auf dem Ruhepuls, dem Alter des Patienten und darauf basierend zu bestimmen, ob der Patient herzfrequenzsenkende Medikamente einnimmt. Die von der Bereitstellungseinheit bereitgestellten Werte der medizinischen Indikatoren würden in diesem Fall also insbesondere den Ruhepuls und das Alter des Patienten umfassen, und zudem eine Angabe darüber, ob der Patient herzfrequenzsenkende Medikamente einnimmt. Statt des Alters des Patienten kann, falls bekannt, auch der Maximalpuls des Patienten als Indikator bereitgestellt werden und als Basis für die Bestimmung der Herzfrequenz-Schwellwerte dienen. Die prähabilitativen Maßnahmen können dann eine regelmäßige Durchführung eines Intervalltrainings über einen vorbestimmten Zeitraum umfassen, wobei die Intervalle des Intervalltrainings hinsichtlich ihrer Intensität durch die bestimmten Herzfrequenz-Schwellwerte bestimmt sind.

Zur Bestimmung der Länge des Zeitraums, über den das regelmäßige Intervalltraining durchzuführen ist, können als weitere medizinische Indikatoren eine oder mehrere Angaben darüber bereitgestellt werden, ob der Patient, wenn er ein Krebspatient ist, eine benigne Indikation aufweist oder der geplante medizinische Eingriff eine neoadjuvante Behandlung darstellt. Ist eines dieser beiden Kriterien erfüllt, kann für Krebspatienten ein längerer prähabilitativer Zeitraum vorgesehen sein, das heißt, ein längerer Zeitraum, über den das kardiovaskuläre Training durchzuführen ist. Hierbei ist das Wort "länger" im Verhältnis zu jenem Zeitraum zu verstehen, der für Patienten vorgesehen ist, die die beiden Kriterien nicht erfüllen.

Die vorgesehene Dauer einer jeweiligen Trainingseinheit kann durch einen festen Wert vorbestimmt sein, im Falle eines Intervalltrainings ebenso die Dauer der einzelnen Intervalle. Bevorzugt ist für jedes Training eine Aufwärmphase und eine Cool-Down-Phase vorgesehen, deren Dauer ebenfalls fest und vorbestimmt sein kann.

In einer beispielhaften Ausführungsform werden die Herzfrequenz-Schwellwerte mittels der Karvonen-Formel bestimmt. Diese Formel ist bekannt und hat sich durch ihre pragmatische Orientierung an der Herzfrequenzreserve, also der Differenz zwischen maximaler Herzfrequenz und der Herzfrequenz in Ruhe (Ruhepuls), als zweckmäßig herausgestellt. Um die von der Karvonen-Formel vorgesehenen Herzfrequenzen für intensives bzw. extensives Ausdauertraining werden vorzugsweise Herzfrequenzzonen definiert, innerhalb derer die Herzfrequenz des Patienten während der Durchführung des jeweiligen Trainingsintervalls liegen sollte. Die Herzfrequenzzonen, die auch als Herzfrequenzbereiche bezeichnet werden könnten, können in ihrer Breite und/oder in ihrer Lage abhängig davon gewählt werden, ob der Patient herzfrequenzsenkende Medikamente nimmt. Beispielsweise kann der Herzfrequenzbereich für intensive Intervalle eine untere Grenze aufweisen, die, wenn der Patient herzfrequenzsenkende Medikamente einnimmt, abgesenkt ist, und der Herzfrequenzbereich für extensive Intervalle eine obere Grenze, die in diesem Fall abgesenkt ist. Der Herzfrequenzbereich für extensive Intervalle und jener für intensive Intervalle können aneinandergrenzen, sodass zwischen ihnen keine "Lücke" von Herzfrequenzen entsteht. Wie oben erwähnt, kann das Intervalltraining auch eine Aufwärmphase und eine Auskühlphase bzw. Cool-Down-Phase aufweisen. Während dieser liegt die Herzfrequenz des Patienten unterhalb der für die intensiven Intervalle vorgesehenen Herzfrequenzen, bevorzugt im Bereich der extensiven Intervalle.

Wie oben erwähnt, werden basierend auf den bereitgestellten Werten von medizinischen Indikatoren sowohl eine Risikoklasse als auch prähabilitative Maßnahmen bestimmt. Dabei können die medizinischen Indikatoren unterteilt sein in solche, die zur Bestimmung der Risikoklasse dienen, und solche, die zur Bestimmung der Parameterwerte für die prähabilitativen Maßnahmen dienen. Ebenso kann allerdings vorgesehen sein, dass einer oder mehrere der medizinischen Indikatoren sowohl für die Bestimmung der Risikoklasse als auch für die Bestimmung der Werte der Mehrzahl von Parametern der prähabilitativen Maßnahmen verwendet werden. Die Risikoklasse kann zu Beginn der prähabilitativen Maßnahmen bestimmt werden, zu vorgegebenen oder beliebigen Zeitpunkten während der prähabilitativen Maßnahmen und/oder am Ende der prähabilitativen Maßnahmen. Durch Vergleich der zu verschiedenen Zeitpunkten bestimmten Risikoklassen kann eine Entwicklung des prähabilitativen Zustands des Patienten bestimmt werden, die als gutes Indiz für eine Wirkung der prähabilitativen Maßnahmen interpretiert werden kann. Zu dem gleichen Zweck können zusätzlich auch zu den verschiedenen Zeitpunkten ermittelte Werte der medizinischen Indikatoren selbst verglichen werden.

Zu den bereitgestellten medizinischen Indikatoren können auch solche zählen, die für eine subjektive Einschätzung des Patienten über seine Lebensqualität indikativ sind. Eine solche subjektive Einschätzung des Patienten kann, insbesondere wenn der Patient ein Krebspatient ist, basierend auf einer Eingabe des Patienten in Bezug auf Fragen des bekannten Fragenkatalogs "QLQ-C30" der "European Organization for Research and Treatment of Cancer" (EORTC) bestimmt werden. Zudem kann es vorteilhaft sein, dass die medizinischen Indikatoren einen oder mehrere Indikatoren für ein Abschneiden des Patienten in einem körperlichen Belastungstest umfassen. Der körperliche Belastungstest kann insbesondere der bekannte 6-Minuten-Gehtest sein, d.h. ein Test, wie weit der Patient innerhalb von 6 Minuten gehen kann. Der eine oder die mehreren Indikatoren für das Abschneiden des Patienten in dem körperlichen Belastungstest können automatisiert im Wege der Anleitung und Überwachung des Belastungstests durch die oben genannte Anwendung auf dem Endgerät des Nutzers erfasst werden. Beispielsweise kann das Endgerät einen Positionssensor und Anzeigemittel aufweisen, die den Nutzer zur Durchführung des 6-Minuten-Gehtests veranlassen können, wobei der Positionssensor während der Durchführung des Gehtests die dabei zurückgelegte Strecke misst. Zusammen mit einer während der Durchführung des Tests gemessenen Herzfrequenz des Patienten, oder auch ohne diese, kann die zurückgelegte Strecke als Indikator für das Abschneiden des Patienten in dem Test bereitgestellt werden.

Die letztgenannten Indikatoren, das heißt, Indikatoren, die für eine subjektive Einschätzung des Patienten über seine Lebensqualität indikativ sind, und solche, die für ein Abschneiden des Patienten in einem körperlichen Belastungstest indikativ sind, werden bevorzugt erfasst, nachdem durch Bestimmen der Risikoklasse und Parameterwerte für die prähabilitativen Maßnahmen basierend auf den zuvor genannten Indikatoren die Maßnahmen eingeleitet wurden, also beispielsweise individuell durch den Patienten nach einem initialen Termin beim Arzt. Wie oben erwähnt, können auch diese Indikatoren beispielsweise zu Beginn und zum Ende des für die prähabilitativen Maßnahmen vorgesehenen Zeitraums erfasst werden, um daraus eine Entwicklung des Zustands des Patienten zu erkennen. Auf diese Weise kann einem behandelnden Arzt eine noch weiter verbesserte Entscheidungshilfe dafür gegeben werden, ob der Patient zu dem medizinischen Eingriff zugelassen werden sollte.

Das System kann ferner eine Maßnahmenüberwachungseinheit aufweisen, die eingerichtet ist, die prähabilitativen Maßnahmen zu überwachen durch a) Erfassen von für eine tatsächliche Durchführung der prähabilitativen Maßnahmen durch den Patienten indikativen Durchführungskennwerten und b) Bestimmen von für eine Durchführungsqualität indikativen Qualitätswerten basierend auf den Durchführungskennwerten und vorbestimmten Schwellwerten für die Durchführungskennwerte. Es können also Durchführungskennwerte erfasst werden, die indikativ für eine tatsächliche Durchführung der prähabilitativen Maßnahmen durch den Patienten sind, wobei "tatsächlich" in Abgrenzung zu einer vorgesehen, "Soll"-Durchführung zu verstehen ist. Im Falle eines kardiovaskulären Intervallteils können die Durchführungskennwerte beispielsweise der während des Trainings aufgenommenen Herzfrequenz des Patienten entsprechen, insbesondere einem zeitlichen Verlauf der Herzfrequenz. Auf Basis der Durchführungskennwerte können dann Qualitätswerte bestimmt werden, die indikativ für eine Durchführungsqualität der prähabilitativen Maßnahmen. Im Falle eines kardiovaskulären Intervalltrainings können die Qualitätswerte beispielsweise eine Auskunft darüber geben, inwieweit der Patient innerhalb der vorgegebenen Herzfrequenzzonen trainiert hat. Die Qualitätswerte können insbesondere basierend auf den Durchführungskennwerten und vorbestimmten Schwellwerten bestimmt werden, wobei die Schwellwerte beispielsweise vorgeben, für welche Werte der Durchführungskennwerte die Qualität der tatsächlichen Durchführung einer jeweiligen prähabilitativen Maßnahme, d.h. etwa eines Bestandteils, einer Einheit oder einer Wiederholung der prähabilitativen Maßnahmen, als gut, ausreichend oder ungenügend verstanden wird. Anhand der Qualitätswerte kann sowohl der Nutzer als auch der behandelnde Arzt oder medizinisches Personal nachvollziehen, ob die prähabilitativen Maßnahmen erfolgsversprechend durchgeführt werden oder wurden. Gegebenenfalls kann auch korrigierend eingegriffen werden, wenn sich die prähabilitativen Maßnahmen anhand der Qualitätswerte als überfordernd oder unterfordernd für den Patienten herausstellen.

Die Maßnahmenüberwachungseinheit kann ferner eingerichtet sein, eine Nutzereingabe zu erfassen, die indikativ dafür ist, ob der Patient bei der Durchführung einer prähabilitativen Maßnahme medizinische Symptome erlebt hat, und basierend auf der Nutzereingabe in Bezug auf die durchgeführte prähabilitative Maßnahme einen oder mehrere Symptomkennwerte zu bestimmen. Es können also nicht nur Kennwerte erfasst werden, die die tatsächliche Durchführung einer prähabilitativen Maßnahme beschreiben und damit eine Einschätzung der Durchführungsqualität erlauben, sondern zudem Kennwerte, die Auskunft über eine Wechselwirkung zwischen der Durchführung der prähabilitativen Maßnahme und einem Krankheitsbild des Patienten geben, wegen dessen der medizinische Eingriff geplant ist, oder über den allgemeinen körperlich-gesundheitlichen Zustand des Patienten. Hierzu hat es sich als zweckmäßig herausgestellt, Antworten des Nutzers selbst auf vorgegebene Fragen nach während der Durchführung einer jeweiligen prähabilitativen Maßnahme erlebten medizinischen Symptomen zu erfassen und die Symptomkennwerte basierend auf den Antworten des Patienten zu bestimmen. Durch die Überwachung von prähabilitativen Maßnahmen anhand der Symptomkennwerte wird das Risiko, dass die prähabilitativen Maßnahmen dem Patienten eher schaden als ihm zu nutzen, wesentlich verringert. Zudem kann noch besser korrigierend in die prähabilitativen Maßnahmen eingegriffen werden.

Die von der Nutzereingabe indizierten medizinischen Symptome können insbesondere eines oder mehrere der folgenden umfassen: Herzstolpern, Brustschmerzen, Kurzatmigkeit, Müdigkeit, Übelkeit, Muskelschmerzen. Der Nutzer kann also beispielsweise über sein bereit oben genanntes Endgerät mit der darauf installierten Anwendung, die ihn bei den prähabilitativen Maßnahmen begleitet, zusätzlich nach jeder Durchführung einer jeweiligen Maßnahme erfassen, ob er Symptome erlebt hat, und wenn ja, ob die Symptome eines oder mehrere der genannten Symptome umfassen.

In einer Ausführungsform ist die Maßnahmenüberwachungseinheit eingerichtet, basierend auf den bestimmten Qualitätswerten und vorzugsweise auch dem einen oder den mehreren bestimmten Symptomkennwerten eine Ausgabeeinheit des Systems dazu zu veranlassen, eine Ausgabe bereitzustellen, die geeignet ist, eine Maßnahmenüberprüfung durch den Arzt herbeizuführen. Statt dem Arzt, das heißt, einem den Patienten behandelnden Arzt, könnte auch eine andere Person die Überwachungsfunktion erfüllen, beispielsweise entsprechend geschultes medizinisches Personal. Die Person kann beispielsweise über ein Endgerät, auf dem eine zu diesem Zweck eingerichtete Anwendung installiert ist, benachrichtigt werden, wenn nach Durchführung einer prähabilitativen Maßnahme eines einer Mehrzahl von überwachten Patienten ein Qualitätswert und/oder ein Symptomkennwert bestimmt worden ist, der anhand vorbestimmter Kriterien eine Maßnahmenüberprüfung für diesen Patienten vorsieht. In diesem Zusammenhang kann das benachrichtigende Endgerät also als Ausgabeeinheit des Systems verstanden werden, wobei sich die Ausgabe auf die Benachrichtigung bezieht. Das Endgerät und die Anwendung können diejenigen sein, über die die zur Initiierung des Maßnahmenzeitraums erforderlichen medizinischen Indikatorwerte erfasst worden sind.

Wie bereits weiter oben erwähnt, können die prähabilitativen Maßnahmen für einen vorgegebenen Zeitraum vorgesehen sein. Dieser Zeitraum kann auch als Maßnahmenzeitraum bezeichnet werden. Wenigstens einer der Mehrzahl von Parametern der prähabilitativen Maßnahmen können den Maßnahmenzeitraum bestimmen, über den hinweg die Maßnahmen von dem Patienten wiederholt auszuführen sind. Beispielsweise kann eine Maßnahme und ein Maßnahmenzeitraum vorgegeben werden, wobei die Maßnahme über den Maßnahmenzeitraum hinweg wiederholt von dem Patienten durchzuführen ist, so dass sich durch die Wiederholung eine Mehrzahl von Maßnahmen ergibt. Es können aber auch verschiedene Maßnahmen vorgegeben sein, die jeweils über den Maßnahmenzeitraum hinweg zu wiederholen sind. Beispielsweise kann zusätzlich zu einem kardiovaskulären Intervalltraining eine andere Art von kardiovaskulärem Training vorgesehen sein, oder aber ein anderes körperliches Training.

Vorzugsweise ist die Bereitstellungseinheit eingerichtet, erste Werte der Mehrzahl medizinischer Indikatoren zu einem ersten Zeitpunkt und zweite Werte der Mehrzahl medizinischer Indikatoren zu einem zweiten Zeitpunkt bereitzustellen, wobei der erste Zeitpunkt einem Beginn und der zweite Zeitpunkt einem Ende des Maßnahmenzeitraums entsprechen, und wobei das System ferner eine Wirkungsbestimmungseinheit aufweist, die eingerichtet ist, basierend auf den ersten Werten und den zweiten Werten der Mehrzahl medizinischer Indikatoren einen oder mehrere Wirkungskennwerte zu bestimmen, die indikativ für eine Wirkung der prähabilitativen Maßnahmen auf das Risiko sind, das der geplante medizinische Eingriff für den Patienten birgt. Die Wirkungskennwerte können beispielsweise angeben, ob sich der Patient hinsichtlich eines jeweiligen medizinischen Indikators verbessert hat, das heißt, ausgehend von diesem Indikator das Risiko verringern konnte, Schaden von dem geplanten medizinischen Eingriff davonzutragen. Statt der Bestimmung von Wirkungskennwerten im eigentlichen Sinne kann also auch lediglich ein Wertevergleich der medizinischen Indikatoren zwischen dem ersten und dem zweiten Zeitpunkt vorgenommen werden, wobei das Vergleichsergebnis zur Beurteilung der Wirkung der prähabilitativen Maßnahmen herangezogen wird.

Vorzugsweise ist die Bereitstellungseinheit ferner eingerichtet, Werte einer Mehrzahl weiterer medizinischer Indikatoren bereitzustellen, wobei die weiteren Indikatoren indikativ für einen Zustand des Patienten nach dem medizinischen Eingriff sind. Dies erlaubt eine Überprüfung des Systems selbst, insbesondere hinsichtlich der Art und Weise, wie es eingerichtet ist, prähabilitative Maßnahmen anhand von medizinischen Indikatoren vorzugeben, und wie die Risikoklasse bestimmt und gegebenenfalls eine darauf basierende Eingruppierung in Risikogruppen vorgenommen wird. Die weiteren medizinischen Indikatoren, die indikativ für den Zustand des Patienten nach dem medizinischen Eingriff sind, können insbesondere angeben, wie der medizinische Eingriff verlaufen ist. In einer Ausführungsform ist die Mehrzahl weiterer medizinischer Indikatoren indikativ für wenigstens eines der folgenden: Eine Diagnose, beispielsweise gemäß ICD-10, eine Therapie, beispielsweise in Form eines OPS-Codes, eine Komplikation, beispielsweise schwerste, während des medizinischen Eingriffs, vorzugsweise in Form eines Clavien-Dindo-Scores, und ob der Patient noch lebt, sowie falls ja, das Datum des letzten Patientenkontakts, und falls nein, den Todeszeitpunkt. Diese weiteren medizinischen Indikatoren sind wiederum an sich bekannt. Die genannte Auswahl wird aber als besonders zweckmäßig erachtet. Sie erlaubt eine unkomplizierte und dennoch umfassende Beurteilung des Erfolgs der prähabilitativen Maßnahmen.

Wie bereits oben erwähnt, kann das System auf einem Server implementiert sein. Alle erfassten Daten, insbesondere die medizinischen Indikatoren, das heißt, die zur Bestimmung der Risikoklasse und der prähabilitativen Maßnahmen verwendeten Indikatoren und die weiteren, nach dem medizinischen Eingriff erfassten Indikatoren, die Durchführungskennwerte, die Qualitätswerte, die Symptomkennwerte, die Wirkungskennwerte, etc. können auf dem Server oder einer damit verbundenen Datenbank gespeichert werden, und zwar für eine Vielzahl von Patienten. Ein behandelnder Arzt oder entsprechend autorisiertes medizinisches Personal kann also Zugriff auf die Daten einer Vielzahl von Patienten haben, um so beispielsweise die Durchführung der prähabilitativen Maßnahmen zu überwachen.

Durch die patientenübergreifende Auswertung der Daten, insbesondere der weiteren medizinischen Indikatoren, kann das System kontinuierlich verbessert werden. Beispielsweise kann die Auswahl der verwendeten medizinischen Indikatoren mit der Zeit verändert werden und die prähabilitativen Maßnahmen angepasst werden.

Vorzugsweise werden die für einen Patienten gesammelten Daten nach Erfassung der Werte der weiteren Indikatoren auf einer separaten Datenbank anonymisiert abgelegt, die mit einem separaten Server verbunden sein kann. Basierend auf den auf diese Weise separat abgelegten Daten kann durch maschinelles Lernen ein Modell trainiert werden, für gegebene Werte der medizinischen Indikatoren eine zugehörige Risikoklasse und zugehörige Parameter prähabilitativer Maßnahmen zu bestimmen. Die Klassifizierungseinheit und die Parametrisierungseinheit können auf das trainierte Modell zugreifen, um die Risikoklasse bzw. die Parameter für neue Patienten folgende Patienten zu bestimmen. Alternativ kann durch statistische Auswertungen der Daten auf dem separaten Server ein regelbasierter Zusammenhang zwischen Werten der medizinischen Indikatoren und der zugehörige Risikoklasse sowie der zugehörigen Parameter prähabilitativer Maßnahmen angepasst werden. Die Klassifizierungseinheit und die Parametrisierungseinheit können also auch manuell entsprechend rekonfiguriert werden. Basierend auf den gesammelten Daten kann insbesondere ermittelt oder "gelernt" werden, wie die Parameter für das prähabilitative Training abhängig auch von der Risikoklasse zu wählen sind, um die Wirkung der prähabilitativen Maßnahmen zu vergrößern, also die Risiken medizinischer Eingriffe weiter zu verringern.

Ein weiterer Aspekt der Erfindung bezieht sich auf ein Endgerät zur Unterstützung eines Patienten bei der Durchführung prähabilitativer Maßnahmen, wobei das Endgerät eingerichtet ist, mit dem oben beschriebenen System zu kommunizieren, und wobei das Endgerät eine Ausgabeeinheit aufweist, die eingerichtet ist, eine Durchführung einer Maßnahme durch den Patienten basierend auf den von der Parametrisierungseinheit des Systems bereitgestellten Parametern durch auditive und/oder visuelle Ausgaben anzuleiten.

In einem weiteren Aspekt bezieht sich die Erfindung auf ein Endgerät zur Unterstützung eines Arztes bei der Beurteilung eines Risikos, das ein geplanter medizinischer Eingriff für einen Patienten birgt, wobei das Endgerät eingerichtet ist, mit dem oben beschriebenen System zu kommunizieren, und wobei das Endgerät eine Ausgabeeinheit aufweist, die eingerichtet ist, zumindest basierend auf der von der Klassifizierungseinheit des Systems bestimmten Risikoklasse auditive und/oder visuelle Ausgaben zu erzeugen.

Die Endgeräte machen sich die gleichen oder ähnliche Vorteile zunutze, wie sie oben hinsichtlich des erfindungsgemäßen Systems beschrieben worden sind. Alternativ oder zusätzlich zu den auditiven und/oder visuellen Ausgaben der Endgeräte sind auch haptische Ausgaben, das heißt, haptische Signale denkbar. Beispielsweise kann der Patient durch ein Display des Endgeräts zu seiner Unterstützung, durch von dem Endgerät erzeugte akustische Signale und/oder durch Vibration des Endgeräts bei der Durchführung der prähabilitativen Maßnahme unterstützt werden.

Die Ausgaben bzw. Signale können im Falle eines kardiovaskulären Trainings beispielsweise basierend auf der Herzfrequenz des Patienten und den vorgegebenen Herzfrequenzzonen erzeugt werden, oder sie können an die Durchführung der prähabilitativen Maßnahmen erinnern.

Die auditiven, visuellen und/oder haptischen Ausgaben oder Signale des Endgeräts zur Unterstützung des Arztes können sich beispielsweise auf eine Nutzeroberfläche einer auf dem Endgerät installierten Anwendung beziehen, über die der Arzt die Durchführung der prähabilitativen Maßnahmen durch seine Patienten überwacht, wobei beispielsweise bei Überschreiten von Durchführungskennwerten und/oder Qualitätswerten über vorbestimmte Schwellwerte akustische Signale erzeugt werden können oder farbliche Markierungen entsprechender Anzeigeelemente der Nutzeroberfläche vorgesehen sein können. Als besonders hilfreiche Darstellungsform hat sich etwa herausgestellt, die Wirkungskennwerte oder ein Ergebnis des Vergleichs zwischen den Werten der medizinischen Indikatoren zwischen Beginn und Ende des Maßnahmenzeitraums farblich kodiert zu präsentieren. Beispielsweise kann abhängig davon, ob ein jeweiliger Indikator sich im Verlaufe des Maßnahmenzeitraums verschlechtert oder verbessert hat, oder ob er im Wesentlichen unverändert geblieben ist, eine jeweils andere Farbe vorgesehen sein, wobei entsprechend farblich gestaltete Anzeigeelemente für jeden der medizinischen Indikatoren in einer gemeinsamen Ansicht präsentiert werden.

Die Erfindung betrifft insbesondere eine Verwendung des Systems und/oder von einem oder beiden der Endgeräte durch einen oder mehrere Patienten bzw. Ärzte zur Unterstützung des jeweiligen Patienten bei der Durchführung prähabilitativer Maßnahmen bzw. zur Unterstützung des jeweiligen Arztes bei der Beurteilung eines Risikos, das ein geplanter medizinischer Eingriff auf einen Patienten birgt.

Gemäß einem weiteren Aspekt bezieht sich die Erfindung auf ein Verfahren zur Verringerung von Risiken medizinischer Eingriffe, wobei das Verfahren ein Bereitstellen von Werten einer Mehrzahl medizinischer Indikatoren umfasst, die indikativ für einen prähabilitativen Zustand eines Patienten sind. Zudem umfasst das Verfahren ein Bestimmen einer Risikoklasse für den Patienten basierend auf den bereitgestellten Werten der Mehrzahl medizinischer Indikatoren, wobei die Risikoklasse indikativ für ein Risiko ist, das ein geplanter medizinischer Eingriff für den Patienten birgt. In einem dritten Schritt werden Werte einer Mehrzahl von Parametern prähabilitativer Maßnahmen für den Patienten basierend auf den Werten der Mehrzahl medizinischer Indikatoren und/oder der Risikoklasse bestimmt, wobei die Maßnahmen geeignet sind, das Risiko, das der geplante medizinische Eingriff für den Patienten birgt, zu senken. Das Verfahren kann insbesondere auf dem oben beschriebenen System ausgeführt werden. Es macht sich die gleichen oder ähnliche Vorteile zunutze, wie sie oben hinsichtlich des Systems beschrieben worden sind.

Zudem bezieht sich die Erfindung in einem Aspekt auf ein Computerprogramm zur Verhinderung von Risiken medizinischer Eingriffe, wobei das Programm Anweisungen aufweist, die geeignet sind, das oben beschriebene System zu veranlassen, das genannte Verfahren auszuführen. Auch das Computerprogramm macht sich die eingangs beschriebenen erfindungsgemäßen Vorteile in gleicher oder ähnlicher Weise zunutze.

Es sollte verstanden werden, dass das System, die Endgeräte, das Verfahren und das Computerprogramm ähnliche und/oder identische bevorzugte Ausführungsformen haben, insbesondere wie sie in den abhängigen Ansprüchen definiert sind.

Es sollte zudem verstanden werden, dass eine bevorzugte Ausführungsform der Erfindung auch jede Kombination von vorliegend offenbarten Ausführungsformen sein kann, insbesondere jede Kombination der abhängigen Ansprüche mit dem jeweiligen unabhängigen Anspruch.

Die oben beschriebenen und weitere Aspekte der Erfindung werden im Folgenden mit Bezug auf einzelne Ausführungsformen, die in den Figuren dargestellt sind, weiter veranschaulicht.

### KURZBESCHREIBUNG DER FIGUREN

Gezeigt sind in:
- Fig. 1: beispielhaft und schematisch ein System zur Verringerung von Risiken medizinischer Eingriffe,
- Fig. 2: beispielhaft und schematisch Geräte, die das System in einer Ausführungsform implementieren,
- Fig. 3: beispielhaft und schematisch medizinische Indikatoren, die zur Initiierung eines Maßnahmenzeitraums erfasst wurden,
- Fig. 4: beispielhaft und schematisch Parameter prähabilitativer Maßnahmen, die aus den medizinischen Indikatoren bestimmt wurden,
- Fig. 5: beispielhaft und schematisch medizinische Indikatoren, die während des Maßnahmenzeitraums erfasst wurden,
- Fig. 6: beispielhaft und schematisch weitere medizinische Indikatoren, die während des Maßnahmenzeitraums erfasst wurden,
- Fig. 7: beispielhaft und schematisch Messwerte, die während der Durchführung einer prähabilitativen Maßnahme durch den Patienten aufgezeichnet werden,
- Fig. 8: beispielhaft und schematisch eine Abfrage von Symptomen, die während der Durchführung der prähabilitativen Maßnahme aufgetreten sind,
- Fig. 9: beispielhaft und schematisch eine Darstellung von Qualitätskennwerten für die Durchführung prähabilitativer Maßnahmen,
- Fig. 10: beispielhaft und schematisch eine Darstellung von Wirkungskennwerten zur Beurteilung einer Wirkung der prähabilitativen Maßnahmen,
- Fig. 11: beispielhaft und schematisch eine Darstellung zur Erfassung von medizinischen Indikatoren für den Zustand eines Patienten nach einem medizinischen Eingriff, und
- Fig. 12: beispielhaft und schematisch ein Verfahren zur Verringerung von Risiken medizinischer Eingriffe.

### DETAILLIERTE BESCHREIBUNG VON AUSFÜHRUNGSFORMEN

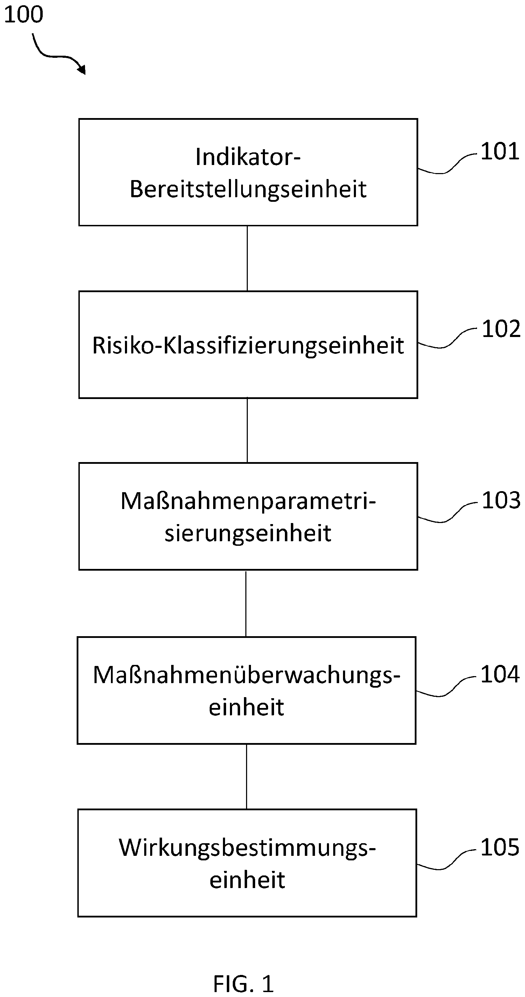

Fig. 1 zeigt beispielhaft und schematisch ein System 100 zur Verringerung von Risiken medizinischer Eingriffe. Das System 100 weist eine Bereitstellungseinheit 101 zum Bereitstellen von Werten einer Mehrzahl medizinischer Indikatoren 10, 11, 12 auf, wie sie im Folgenden noch näher beschrieben werden. Die Indikatoren 10, 11, 12 sind indikativ für einen prähabilitativen Zustand eines Patienten. Die Bereitstellungseinheit 101 stellt die Indikatoren 10, 11, 12 basierend auf entsprechenden Nutzereingaben oder mittels geeigneter Messmittel erfassten Messdaten bereit.

Das System 100 weist ferner eine Klassifizierungseinheit 102 zum Bestimmen einer Risikoklasse 20 für den Patienten basierend auf den Werten der Mehrzahl medizinischer Indikatoren 10, 11, 12 auf. Wie nachfolgend noch ausführlicher erläutert, ist die Risikoklasse 20 indikativ für ein Risiko, das ein geplanter medizinischer Eingriff für den Patienten birgt. Zudem weist das System 100 eine Parametrisierungseinheit 103 zum Bestimmen von Werten einer Mehrzahl von Parametern 30 prähabilitativer Maßnahmen für den Patienten basierend auf den Werten der Mehrzahl medizinischer Indikatoren 10, 11, 12 auf. Dabei sind die Maßnahmen geeignet, das Risiko, das der geplante medizinische Eingriff für den Patienten birgt, zu senken. Für eine beispielhafte Klasse prähabilitativer Maßnahmen werden die Parameter 30 im Folgenden noch näher beschrieben werden.

Das System 100 weist auch eine Maßnahmenüberwachungseinheit 104 auf, die eingerichtet ist, die prähabilitativen Maßnahmen zu überwachen. Dazu erfasst die Maßnahmenübewachungseinheit 104 Durchführungskennwerte 30, 31, die indikativ für eine tatsächliche Durchführung der prähabilitativen Maßnahmen durch den Patienten sind, und bestimmt darauf basierend, und zudem basierend auf vorbestimmten Schwellwerten für die Durchführungskennwerte, Qualitätswerte 40, die für eine Durchführungsqualität der prähabilitativen Maßnahmen durch den Patienten indikativ sind. Auch die Durchführungskennwerte 30, 31 und Qualitätswerte 40 werden nachfolgend noch näher erläutert werden.

Schließlich weist das System 100 auch eine Wirkungsbestimmungseinheit 105 auf, die eingerichtet ist, Wirkungskennwerte 60 zu bestimmen, die indikativ für eine Wirkung der prähabilitativen Maßnahmen auf das Risiko sind, das der geplante medizinische Eingriff für den Patienten birgt. Wie weiter unten im Detail beschrieben, werden die Wirkungskennwerte 60 von der Wirkungsbestimmungseinheit 105 basierend auf ersten und zweiten Werten der Mehrzahl medizinischer Indikatoren 10 bestimmt, wobei die ersten Werte der medizinischen Indikatoren 10 von der Bereitstellungseinheit 101 zu Beginn eines Maßnahmenzeitraums und die zweiten Werte der medizinischen Indikatoren 10 von der Bereitstellungseinheit 101 am Ende des Maßnahmenzeitraums bereitgestellt worden sind.

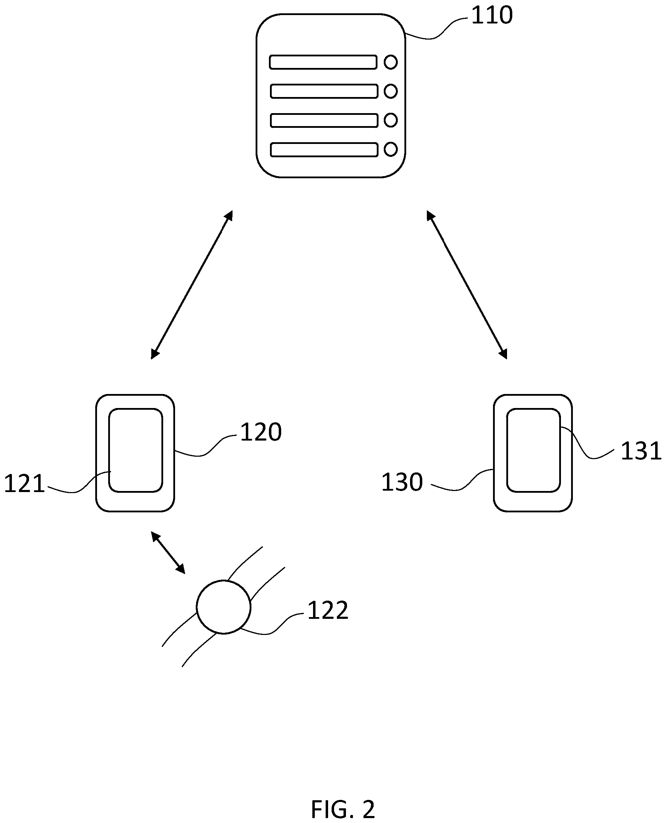

Fig. 2 zeigt beispielhaft und schematisch von dem System 100 beanspruchte Hardwarekomponenten. Diese umfassen ein mobiles Endgerät 130 mit einem Touchscreen 131, das einem Nutzer wie beispielsweise einem behandelnden Arzt oder medizinischem Personal die Eingabe von initial ermittelten Werten medizinischer Indikatoren 10 erlaubt. Die Werte der medizinischen Indikatoren 10 werden dann von dem Endgerät 130, das auch als Teil eines Frontends einer zugrundeliegenden Softwareanwendung verstanden werden könnte, an eine zentrale Rechenstruktur wie beispielsweise einen Server 110 übermittelt, die auch als Backend der Softwareanwendung verstanden werden könnte. Die Übermittlung der Werte der medizinischen Indikatoren 10 von dem Endgerät 130 an den Server 110 geschieht dabei über die Bereitstellungseinheit 101, die beispielsweise einer Schnittstelle zwischen dem Endgerät 130 und dem Server 110 entsprechen könnte. Auf dem Server 110 sind dann die weiteren Einheiten 102 - 105 des Systems 100 implementiert. Über eine weitere Schnittstelle ist der Server 110 mit mobilen Endgeräten 120 einer Vielzahl von Patienten verbunden, wobei auch diese jeweils beispielsweise Touchscreens 121 aufweisen können. Zudem sind die Endgeräte 120 in dem vorliegenden Ausführungsbeispiel mit Messmitteln 122 wie beispielsweise Smartwatches mit Herzfrequenzsensor und Positionssensor gekoppelt. Auch die Schnittstelle zwischen dem Server 110 und einem jeweiligen der Endgeräte 120 kann als Teil der Bereitstellungseinheit 101 aufgefasst werden.

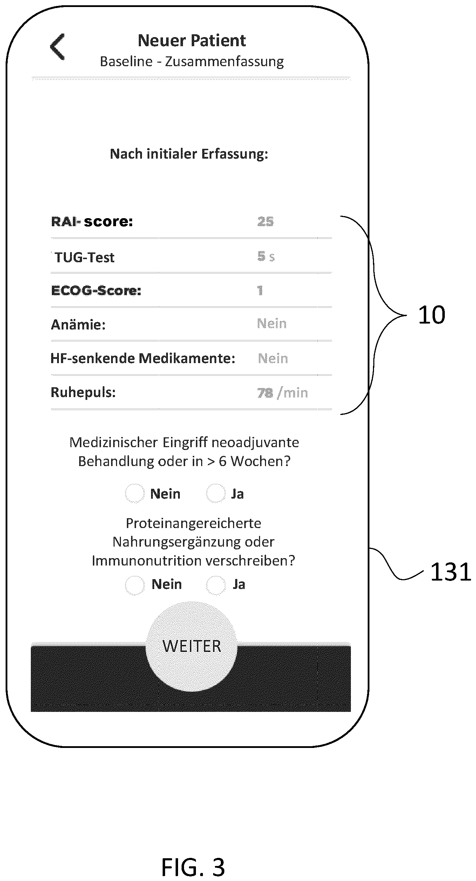

Fig. 3 zeigt beispielhaft und schematisch eine zusammenfassende Darstellung von Werten medizinischer Indikatoren 10, die zur Aufnahme eines Patienten in ein prähabilitatives Maßnahmenprogramm ermittelt worden sind. Die gezeigten Werte werden entweder durch unmittelbare Eingabe in das Endgerät 130 erfasst, oder basierend auf entsprechenden Nutzereingaben in das Endgerät 130 bestimmt. Im gezeigten Beispiel wurde ein RAI-Score, das Ergebnis eines TUG-Tests, ein ECOG-Score und ein Ruhepuls des Patienten erfasst. Zudem wurde erfasst, ob der Patient an Blutarmut leidet, ob er herzfrequenzsenkende Medikamente einnimmt, ob der Patient ein Krebspatient ist, für den eine neoadjuvante Behandlung als medizinischer Eingriff geplant ist oder ob der medizinische Eingriff in mehr als sechs Wochen stattfinden wird, und ob eine proteinangereicherte Nahrungsergänzung oder eine Immunonutrition verschrieben wird.

Der RAI-Score wurde gemäß folgendem Artikel ermittelt: *"*Development and Initial Validation ofthe Risk Analysis Index for Measuring Frailty in Surgical Populations" von D. E. Hall et al. JAMA Surgery 2017; 152(2):175-182. Dabei wurde der Nutzer des Endgeräts 130, also bspw. der Arzt, aufgefordert, über entsprechende Nutzereingaben die Fragen des in dem genannten Artikel vorgeschlagenen Fragenkatalogs für den Patienten zu beantworten, wobei basierend auf den Nutzereingaben, das heißt, auf den Antworten, gemäß dem in dem zitierten Artikel vorgeschlagenen Scoring-System der RAI-Score bestimmt worden ist.

Zudem wurde mit dem Patienten der "Timed up and go (TUG)"-Test durchgeführt und das Ergebnis, das heißt, die von dem Patienten benötigte Zeit eingegeben. Der TUG-Test, wobei TUG für "Timed up and go" steht, ist ein Test der Mobilität einer Person. Dazu setzt sich die Person auf einen Stuhl mit Armlehnen, erhebt sich auf Aufforderung ohne fremde Hilfe, geht anschließend eine Strecke von 3 Metern, kehrt um und zum Stuhl zurück, und setzt sich daraufhin wieder auf dem Stuhl hin. Zur Bewältigung der Strecke darf die Person Hilfsmittel wie beispielsweise eine Gehstütze verwenden. Beginnend mit der Aufforderung aufzustehen wird die Zeit gemessen, bis die Person sich wieder hingesetzt hat, wobei die gemessene Zeit wie folgt in Mobilitätsklassen eingeordnet wird, die vorliegend beispielsweise als TUG-Ergebnis herangezogen werden können:

| | |
|---|---|
| 10 Sekunden | Alltagsmobilität uneingeschränkt |
| 10 - 19 Sekunden | geringe Mobilitätseinschränkung, in der Regel noch ohne Alltagsrelevanz |
| 20 - 29 Sekunden | abklärungsbedürftige, funktionell relevante Mobilitätseinschränkung |
| 30 und mehr Sekunden | ausgeprägte Mobilitätseinschränkung |

Der ECOG-Score wurde ebenfalls unmittelbar eingegeben, vorzugsweise unter Hilfestellung durch Angabe der Kriterien, die zu dem jeweiligen Score-Wert führen. Der ECOG-Status kann beispielsweise gemäß der folgenden Tabelle bestimmt werden, wobei die linke Spalte den jeweils heranzuziehenden numerischen Wert angibt und die rechte Spalte die Kriterien, nach denen sich bestimmt, welcher der Werte für den Patienten angenommen wird.

| | |
|---|---|
| 0 | Der Patient ist asymptomatisch. |
| 1 | Der Patient ist symptomatisch, aber vollständig ambulant. |
| 2 | Der Patient ist symptomatisch, aber nicht vollständig ambulant. Verbringt unter 50 % der Zeit des Tages im Bett. |
| 3 | Der Patient ist symptomatisch und verbringt über 50 % der Zeit des Tages im Bett, ist aber nicht ans Bett gebunden. |
| 4 | Der Patient ist ans Bett gebunden. |
| 5 | Der Patient ist verstorben. |

Ob der Patient unter Blutarmut leidet, wurde automatisiert basierend auf einer Nutzereingabe eines gemessenen Hämoglobinwerts des Patienten ermittelt, wobei ein oder mehrere vordefinierte Grenzwerte für den Hämoglobinwert herangezogen werden können, um aus diesen auf eine etwaige Blutarmut zu schließen.

Die übrigen Angaben, die in Fig. 3 zu sehen sind, wurden unmittelbar eingegeben, beispielsweise unter Rückgriff auf die Patientenakte. Der Ruhepuls könnte auch neu vermessen oder von dem Patienten erfragt worden sein.

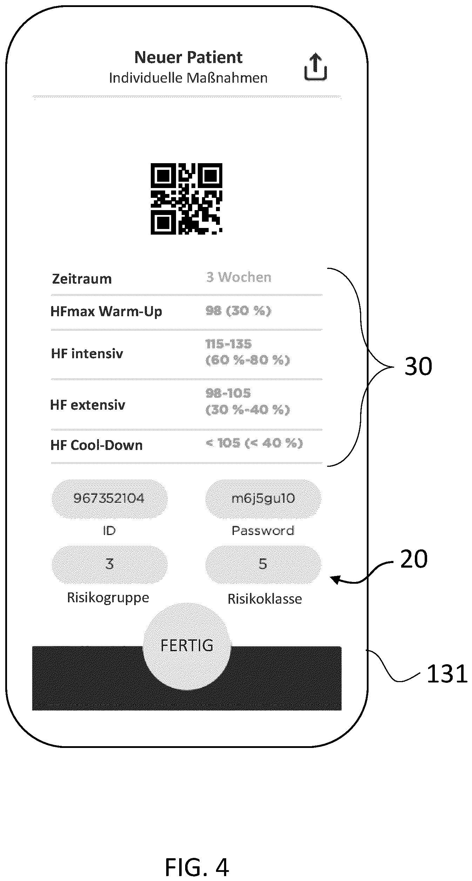

Basierend auf den initial erfassten Angaben, die in Fig. 3 beispielhaft zusammengefasst sind, bestimmt die Klassifizierungseinheit 102 die Risikoklasse 20 für den Patienten, und die Parametrisierungseinheit 103 bestimmt die Parameterwerte 30 für die prähabilitativen Maßnahmen. Diese sind in Fig. 4 beispielhaft und schematisch dargestellt, wobei sich die prähabilitativen Maßnahmen in dem dargestellten Fall auf ein kardiovaskuläres Intervalltraining beziehen. Das Intervalltraining soll, etwa weil der medizinische Eingriff eine nichtneoadjuvante Behandlung eines bösartigen Tumors darstellt und damit ein längerer Aufschub als unverhältnismäßig eingeschätzt wird, über einen Zeitraum von 3 Wochen wiederholt durchgeführt werden, wobei jede Durchführung nach dem gleichen Intervallschema erfolgen soll. Dazu definieren die Parameter 30 jeweils einen Herzfrequenzbereich für eine Aufwärmphase, die extensiven Intervalle, die intensiven Intervalle und eine Auskühlphase, die auch als Cool-Down bezeichnet werden kann. Die Herzfrequenzbereiche für die extensiven und die intensiven Intervalle umschließen die jeweilige Herzfrequenz, die gemäß der Karvonen-Formel vorgesehen ist. Die Herzfrequenzbereiche können beispielsweise gemäß folgender Tabelle bestimmt werden, wobei HF für Herzfrequenz und HFR fürdie Herzfrequenzreserve steht, wobei mit Ruhepuls H0 und maximaler Herzfrequenz HFmax [in Schlägen pro Minute] ≈ 220 - Alter [in Jahren] die Relation HFR = HFmax - H0 gilt:

| | Falls der Patient keine herzfrequenzsenkenden Medikamente einnimmt: | Falls der Patient herzfrequenzsenkende Medikamente einnimmt: |
|---|---|---|
| Warm-Up | HF < H0 + 60 % HFR | HF < H0 + 50 % HFR |
| Intensive Intervalle | H0 + 60 % HFR ≤ HF < H0 + 85 % HFR | H0 + 50 % HFR ≤ HF < H0 + 85 % HFR |
| Extensive Intervalle | H0 + 30 % HFR ≤ HF < H0 + 60 % HFR | H0 + 30 % HFR ≤ HF < H0 + 50 % HFR |
| Cool-Down | HF < R0 + 60 % HFR | HF < H0 + 50 % HFR |

Wie in Fig. 4 dargestellt, können die Herzfrequenzbereiche auch in Relation zu HFmax angegeben werden. Nicht in Fig. 4 zu sehen sind vordefinierte Parameterwerte, die unabhängig von den Angaben aus Fig. 3 sind. Hierzu gehören die Dauer eines einzelnen Intervalltrainings (bspw. 37 Minuten), die Dauer und Anzahl der Intervalle (bspw. 5 Minuten Aufwärmen, 2-minütige intensive und 3-minütige extensive Intervalle im Wechsel, 5 Minuten Cool-Down) sowie die Häufigkeit, mit der das Training wiederholt werden soll (bspw. zwischen 0 bis 4 Trainings in der Woche).

Die Risikoklasse 20 wird beispielsweise wie folgt bestimmt: Für TUG-Testergebnisse unter 9 Sekunden wird ein TUG-Teilscore von 0 angenommen, für TUG-Testergebnisse gleich oder über 9 Sekunden ein TUG-Teilscore von 1. Für ECOG-Werte von 0 wird ein ECOG-Teilscore von 0 angenommen, für ECOG-Werte von 1 oder 2 ein ECOG-Teilscore von 1 und für ECOG-Werte von 3 ein ECOG-Teilscore von 2. Für Hämoglobinwerte gleich oder oberhalb von 13 g/dl wird ein Hämoglobin-Teilscore von 0 angenommen, wobei für Frauen ein Hämoglobinwert von 12 g/dl statt 13 g/dl bei Männern als Grenzwert angesetzt wird. Für Hämoglobinwerte unterhalb von 13 g/dl (für Männer) bzw. 12 g/dl (für Frauen) wird ein Hämoglobin-Teilscore von 2 angesetzt. Für RAI-Werte unterhalb von 26 wird ein RAI-Teilscore von 0 angesetzt, für RAI-Werte gleich oder oberhalb von 26 ein RAI-Teilscore von 1. Dann werden die vier Teilscores (das heißt, der TUG-Teilscore, der ECOG-Teilscore, der RAI-Teilscore und der Hämoglobin-Teilscore) addiert, wobei die Summe als Risikoklasse 20 festgesetzt wird. Optional kann basierend auf der Risikoklasse 20 noch eine Eingruppierung von Patienten in Risikogruppen vorgenommen werden, wie in Fig. 4 zu sehen. Dazu werden Patienten mit einer Risikoklasse von 0 oder 1 als Niedrigrisikopatienten eingestuft und Patienten mit einer Risikoklasse gleich oder oberhalb von 2 als Hochrisikopatienten.

Mit oder nach Bestimmung der Parameter 30 für die prähabilitativen Maßnahmen, die in diesem Fall beispielsweise die Schwellwerte für die Herzfrequenzbereiche des Intervalltrainings umfassen können, und nach der Bestimmung der Risikoklasse 20, kann die Aufnahme des Patienten in das Maßnahmenprogramm abgeschlossen werden. Der Patient kann in der Folge über sein eigenes Endgerät 120 einen eigenen Zugang zu dem System 100, insbesondere zu den Parametern 30 und zu Funktionen zur Erfassung von Daten erhalten, mittels derer die Maßnahmen überwacht werden können. Wie in Fig. 4 dargestellt, kann der Zugang für den Patienten beispielsweise über einen QR-Code und ein Initialpasswort vermittelt werden, die von dem Endgerät 130 bzw. dessen Bildschirm 131 angezeigt werden. Durch Scannen des QR-Codes mit seinem Endgerät 120 kann der Patient dann Zugriff auf eine auf Patienten zugeschnittene Anwendungskomponente des Systems 100 erlangen, wobei er sich in dieser (bspw. in einem in dieser für ihn bereits angelegten Patientenkonto) initial mit dem Initialpasswort anmelden kann.

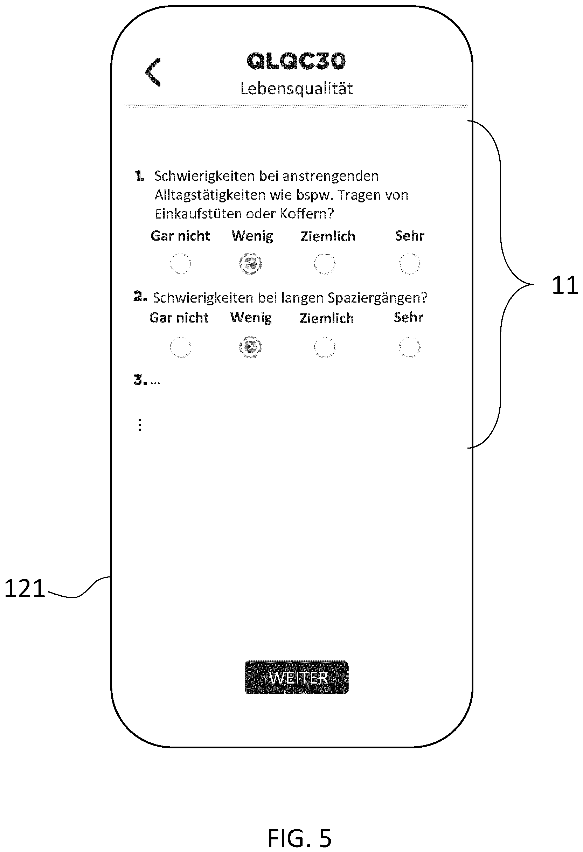

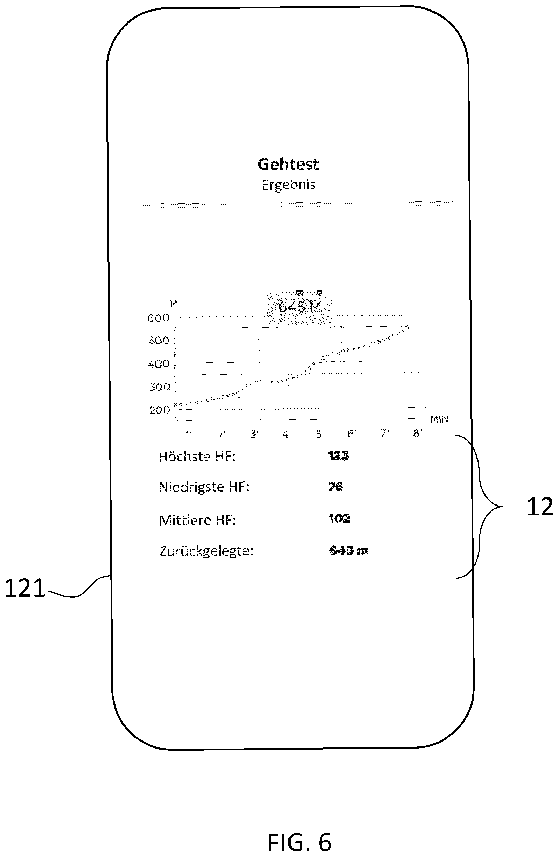

Zu den für den Patienten zugänglichen Funktionen kann beispielsweise der in Fig. 5 gezeigte Fragenkatalog gehören, der zur Erfassung von medizinischen Indikatoren 11 dient, die für eine subjektive Einschätzung des Patienten über seine Lebensqualität indikativ sind. In dem gezeigten beispielhaften Fall ist der Fragenkatalog gemäß QLQ-C30 ausgestaltet. Wie in Fig. 6 gezeigt, kann der Patient über seinen Zugang zu dem System 100 auch zur Durchführung eines 6-minütigen Gehtests angeleitet werden, dessen Ergebnis in Fig. 6 beispielhaft zusammengefasst ist. Vorzugsweise wird der Gehtest über einen Positionssensor ausgewertet, der in dem Endgerät 120 oder einem anderen Messmittel 122 wie beispielsweise einer Smartwatch integriert sein kann, das der Patient während der Durchführung des Gehtests bei sich trägt. Zu den medizinischen Indikatoren 12, die von der Durchführung des Gehtests abgeleitet werden können, kann insbesondere die während der vorgegebenen Zeit von 6 Minuten von dem Patienten zurückgelegte Strecke festgehalten werden. Stellt der Patient während des Maßnahmenzeitraums medizinische Indikatoren wie die in Fig. 5 und Fig. 6 gezeigten Antworten 11 und Gehtest-Ergebnisse 12 zur Verfügung, kann der prähabilitative Fortschritt des Patienten noch während des Maßnahmenzeitraums überwacht werden.

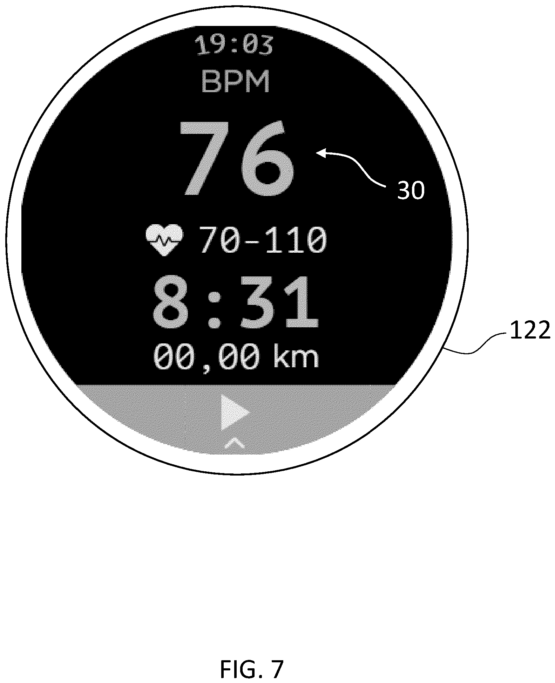

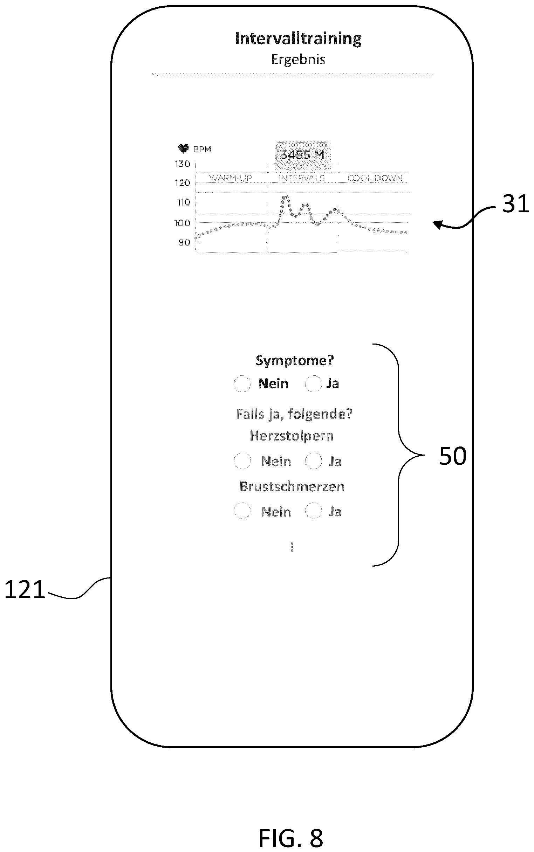

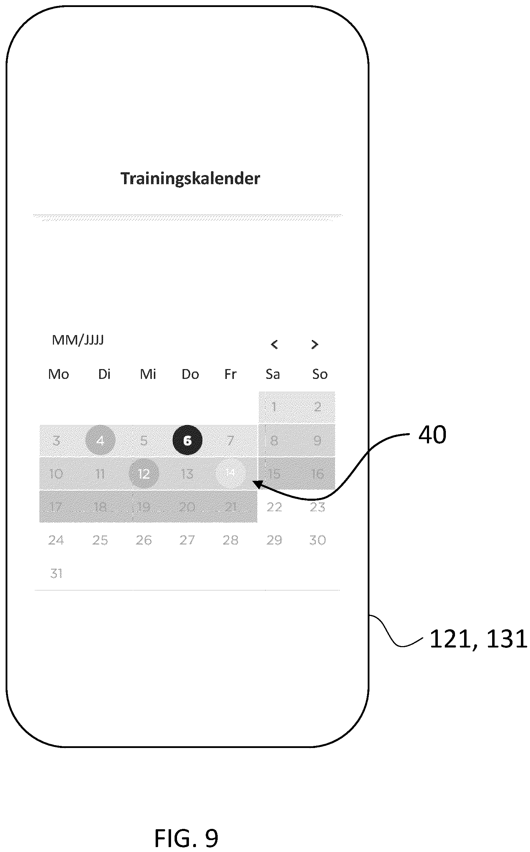

Fig. 7 zeigt beispielhaft und schematisch eine Darstellung, die den Patienten bei der Durchführung einer Intervalltrainingseinheit unterstützen kann. Die Darstellung kann beispielsweise auf dem Display eines Messmittels 122 zum Messen der Herzfrequenz 30 zur Anzeige gebracht werden, wobei das Messmittel vorzugsweise eine Smartwatch oder ein ähnliches "wearable device" ist. Wie in Fig. 7 gezeigt, kann die Darstellung auch einen gerade vorgesehenen Herzfrequenzbereich, eine während der Intervalltrainingseinheit vergangene Zeit sowie eine zurückgelegte Strecke umfassen. Die momentane Herzfrequenz 30 sowie ihr zeitlicher Verlauf 31 bilden Durchführungskennwerte, die indikativ für die tatsächliche Durchführung der prähabilitativen Maßnahme sind. Zusammen mit den Parametern 30, insbesondere den Herzfrequenzbereichen, in denen das Training stattfinden soll, dienen sie der Maßnahmenüberwachungseinheit 104 zur Bestimmung von für die Durchführungsqualität der Maßnahmen indikativen Qualitätswerten 40. Die Qualitätswerte 40 können beispielsweise farbcodiert im Rahmen einer Kalenderdarstellung angezeigt werden, die sowohl für den Patienten als auch für den Nutzer des Endgeräts 130, also beispielsweise den behandelnden Arzt, einsehbar sein kann. Die Kalenderansicht ist in Fig. 9 beispielhaft und schematisch gezeigt. Neben den Qualitätswerten 40, die beispielsweise als Indiz dafür verstanden werden können, inwieweit der Patient innerhalb der vorgegebenen Herzfrequenzbereiche trainiert hat, kann es insbesondere auch von Bedeutung sein, während der Durchführung der Maßnahmen aufgetretene Symptome zu erfassen. Wie in Fig. 8 beispielhaft und schematisch gemeinsam mit dem Verlauf 31 der Herzfrequenz dargestellt, kann zu diesem Zweck nach Abschluss eines jeweiligen Intervalltrainings ein Fragebogen vorgesehen sein, den der Patient aufgefordert wird auszufüllen. Die entsprechenden Nutzereingaben 50 können der Maßnahmenüberwachungseinheit 104 zur Bestimmung von Symptomkennwerten dienen. Besonders relevante Symptome, die die Aufmerksamkeit eines behandelnden Arztes erfordern können, sind beispielsweise Herzstolpern, Brustschmerzen, Kurzatmigkeit, Müdigkeit, Übelkeit und Muskelschmerzen, die während der Durchführung eines kardiovaskulären Intervalltrainings auftreten, insbesondere, wenn dies wiederholt der Fall ist. Die Maßnahmenüberwachungseinheit 104 kann eingerichtet sein, basierend auf den bestimmten Qualitätswerten 40 und den Symptomkennwerten 50 über eine entsprechende Ausgabeeinheit wie den Bildschirm 131 des Endgeräts 130 Warnhinweise bereitzustellen, die den behandelnden Arzt auf eine notwendige Überprüfung der Maßnahmen aufmerksam machen sollen.

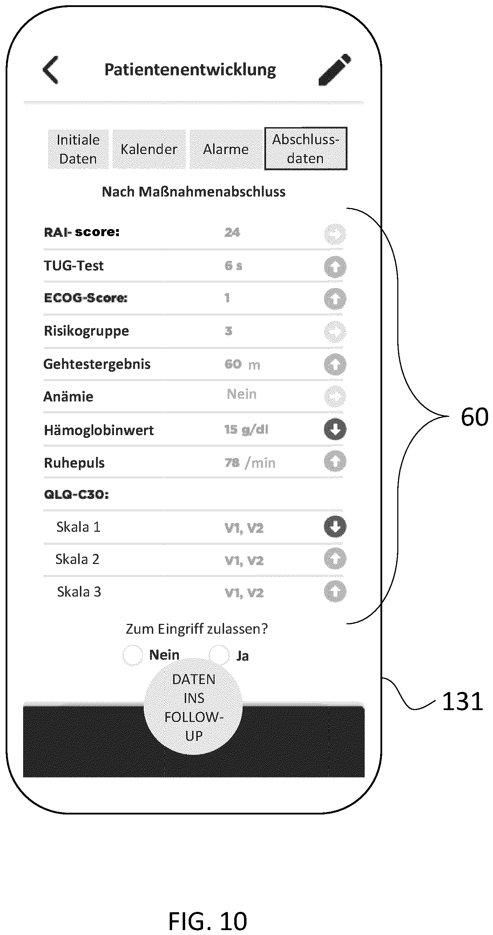

Am Ende des Maßnahmenzeitraums, im vorliegenden Beispiel also etwa nach den in Fig. 4 als Teil der Parameter 30 gezeigten drei Wochen, in denen das vorgeschriebene Intervalltraining wiederholt auszuführen ist, werden erneut Werte für die initial, das heißt, vor Maßnahmenbeginn erfassten medizinischen Indikatoren 10 erfasst und die daraus resultierende Risikoklasse 20 oder eine entsprechende Risikogruppe bestimmt. Wie in Fig. 10 beispielhaft und schematisch gezeigt, können sich die zum Ende des Maßnahmenzeitraums erfassten Werte der medizinischen Indikatoren 10 von den initial erfassten Werten unterscheiden. Üblicherweise werden sie dies auch tun. Gleiches gilt daher für die Risikoklasse oder -gruppe. Durch einen Vergleich der Werte der medizinischen Indikatoren 10 und der Risikoklasse oder -gruppe zwischen Beginn und Ende des Maßnahmenzeitraums kann die Wirkungsbestimmungseinheit 105 Wirkungskennwerte 60 bestimmen, die in Fig. 10 gemäß einem dreiteiligen Farbschema angezeigt werden. Die drei Farben entsprechen der Unterscheidung in solche Indikatoren, deren Wert sich aus körperlich-gesundheitlicher Sicht verbessert hat, solche Indikatoren, deren Wert im Wesentlichen gleichgeblieben ist und solche, deren Wert sich aus körperlich-gesundheitlicher Sicht verschlechtert hat. In Fig. 10 ist dies auch durch mit entsprechenden Pfeilen angegebene Richtungen (oben, zur Seite, nach unten) verdeutlicht.

Wurden im Maßnahmenzeitraum mehrmals, insbesondere zu Beginn und am Ende des Maßnahmenzeitraums, der 6-Minuten-Gehtest durchgeführt sowie Antworten auf die Fragen des QLQ-C30-Fragenkatalogs gegeben, können auch diesbezüglich Wirkungskennwerte 60 bestimmt werden. In Fig. 10 sind auch für diese Wirkungskennwerte entsprechend farblich markierte Pfeilsymbole gezeigt.

Anhand der Darstellung gemäß Fig. 10 kann der behandelnde Arzt entscheiden, ob der Patient zu dem geplanten medizinischen Eingriff zugelassen werden sollte oder nicht. Ist dies nicht der Fall, kann in Betracht gezogen werden, einen weiteren Maßnahmenzeitraum einzuleiten, wobei zur Bestimmung der Parameter 30 der prähabilitativen Maßnahmen in dem weiteren Maßnahmenzeitraum als Grundlage die Werte der medizinischen Indikatoren 10 verwendet werden können, die am Ende des ersten Maßnahmenzeitraums erfasst worden sind.

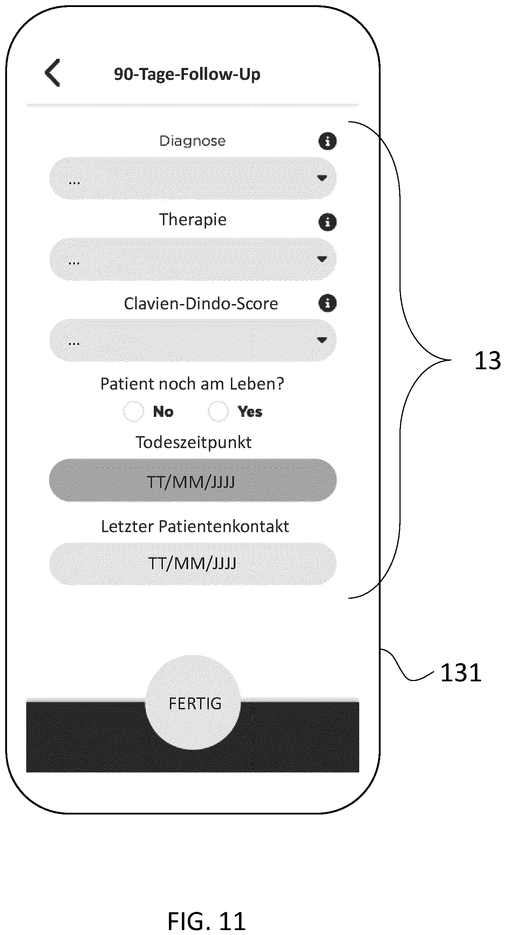

Wird der medizinische Eingriff durchgeführt, kann zu einem späteren Zeitpunkt eine Gesamtbewertung vorgesehen sein. Wie in Fig. 11 beispielhaft und schematisch gezeigt, kann diese etwa 90 Tage nach dem medizinischen Eingriff erfolgen. Dazu können über das Endgerät 130 weitere medizinische Indikatoren 13 bereitgestellt werden, wobei diese weiteren medizinischen Indikatoren 13 indikativ für einen Zustand des Patienten nach dem medizinischen Eingriff sind. Als zweckmäßig haben sich dafür herausgestellt: eine Diagnose, insbesondere gemäß ICD-10, eine Therapie, insbesondere in Form eines OPS-Codes, Indikatoren für während des medizinischen Eingriffs aufgetretene Komplikationen, wobei insbesondere schwerste Komplikationen von Relevanz sind, und wobei der Indikator sich etwa auf einen Clavien-Dindo-Score beziehen kann, und ob der Patient noch lebt. Falls der Patient noch lebt, sollte das Datum des letzten Patientenkontakts hinterlegt werden, anderenfalls der Todeszeitpunkt.

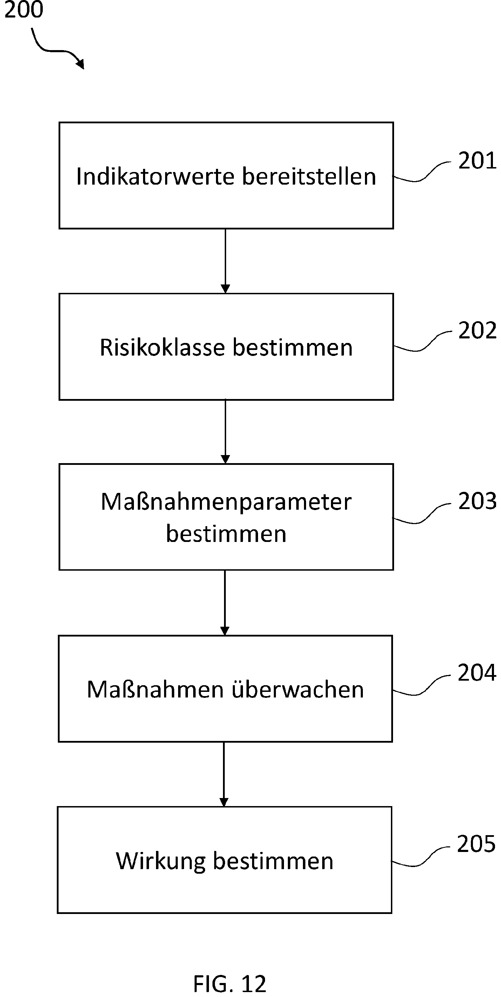

Fig. 12 zeigt beispielhaft und schematisch ein Verfahren 200 zur Verringerung von Risiken medizinischer Eingriffe, wie es zuvor mit Bezug auf das System 100 beschrieben worden ist. Das Verfahren 200 umfasst ein Bereitstellen 201 von Werten einer Mehrzahl medizinischer Indikatoren 10, 11, 12, die indikativ für einen prähabilitativen Zustand eines Patienten sind, ein Bestimmen 202 einer Risikoklasse 20 für den Patienten basierend auf den bereitgestellten Werten der Mehrzahl medizinischer Indikatoren 10, 11, 12, wobei die Risikoklasse indikativ für ein Risiko ist, das ein geplanter medizinischer Eingriff für den Patienten birgt, und ein Bestimmen 203 von Werten einer Mehrzahl von Parametern 30 prähabilitativer Maßnahmen für den Patienten basierend auf den Werten der Mehrzahl medizinischer Indikatoren 10, 11, 12 und/oder der Risikoklasse 20, wobei die Maßnahmen geeignet sind, das Risiko, das der geplante medizinische Eingriff für den Patienten birgt, zu senken.

Zudem umfasst das Verfahren 200 ein Überwachen 204 der prähabilitativen Maßnahmen durch a) Erfassen von für eine tatsächliche Durchführung der prähabilitativen Maßnahmen durch den Patienten indikativen Durchführungskennwerten 30, 31 und b) Bestimmen von für eine Durchführungsqualität indikativen Qualitätswerten 40 basierend auf den Durchführungskennwerten 30, 31 und vorbestimmten Schwellwerten für die Durchführungskennwerte 30, 31. Das Verfahren 200 umfasst zudem ein Bestimmen 205 von einem oder mehreren Wirkungskennwerten 60, die indikativ für eine Wirkung der prähabilitativen Maßnahmen auf das Risiko sind, das der geplante medizinische Eingriff für den Patienten birgt, wobei der mindestens eine Wirkungskennwert 60 basierend auf ersten Werten der Mehrzahl medizinischer Indikatoren 10 und zweiten Werten der Mehrzahl medizinischer Indikatoren 10 bestimmt wird, wobei die ersten Werte zu einem ersten Zeitpunkt und die zweiten Werte zu einem zweiten Zeitpunkt bereitgestellt worden sind, wobei der erste Zeitpunkt einem Beginn eines Zeitraums der prähabilitativen Maßnahmen und der zweite Zeitpunkt einem Ende des Zeitraums der prähabilitativen Maßnahmen entspricht. Die Wirkung kann, wie in Fig. 10 gezeigt, beispielsweise in Form einer Tendenz bestimmt werden, das heißt, angeben, inwieweit sich ein Wert eines jeweiligen medizinischen Indikators 10 über den Maßnahmenzeitraum hinweg verändert hat. Zusätzlich oder alternativ kann ein Wirkungskennwert 60 die Risikoklasse 20, eine entsprechende Eingruppierung in eine Niedrigrisiko- oder eine Hochrisiko-Patientengruppe und/oder eine Veränderung derselben über den Maßnahmenzeitraum hinweg angeben.

Insoweit im Vorangegangenen von der Verwendung einer bestimmten Auswahl medizinischer Indikatoren ausgegangen worden ist, sei klargestellt, dass dies nicht zwingend ist und auch andere Indikatorkombinationen verwendet werden könnten. Zudem wurde oben beispielhaft eine konkrete Berechnungsmethode für die Parameter eines prähabilitativen Intervalltrainings und der Risikoklasse vorgestellt, obwohl auch andere Berechnungsmethoden möglich wären, ebenso wie eine andere Gruppierung von Risikoklassen in Risikogruppen denkbar wäre. Selbstverständlich ist auch die konkret verwendete Hardware, die in Fig. 2 gezeigt ist, nicht die einzig mögliche. Stattdessen könnte beispielsweise die Bestimmung der Parameter für die prähabilitativen Maßnahmen und der Risikoklasse auch lokal auf einem der Endgeräte 120 oder auch auf beiden erfolgen. Zudem sind keine mobilen Endgeräte erforderlich, sondern es könnten auch ortsgebundene Endgeräte verwendet werden, solange die Bereitstellung der benötigten Daten gewährleistet ist.

Auch andere Variationen der offenbarten Ausführungsformen können von dem Fachmann durch ein Studium der Figuren, der Beschreibung und der angehängten Ansprüche verstanden und umgesetzt werden.

In den Ansprüchen schließen die Wörter "aufweisen" und "umfassen" nicht andere Elemente oder Schritte aus, und der unbestimmte Artikel "ein" schließt eine Mehrzahl nicht aus.

Eine einzelne Einheit oder Vorrichtung kann die Funktionen mehrerer Elemente erfüllen, die in den Ansprüchen aufgeführt sind. Die Tatsache, dass einzelne Funktionen und Elemente in unterschiedlichen abhängigen Ansprüchen aufgeführt sind, bedeutet nicht, dass nicht auch eine Kombination dieser Funktionen oder Elemente vorteilhaft verwendet werden könnte. Vorgänge wie das Bereitstellen von Werten medizinischer Indikatoren, das Bestimmen einer Risikoklasse, von Parameterwerten, Kennwerten oder Qualitätswerten, die von einer oder mehreren Einheiten oder Geräten umgesetzt werden, können ebenso von jeder anderen Zahl von Einheiten oder Geräten umgesetzt werden. Diese Vorgänge können durch Programmcodemittel eines Computerprogramms und/oder in Form von dedizierter Hardware implementiert sein. Ein Computerprogrammprodukt kann auf einem geeigneten Medium, wie beispielsweise einem optischen Speichermedium oder einem Festkörperspeicher, gespeichert und/oder verteilt werden, gemeinsam mit oder als Teil von anderer Hardware zur Verfügung gestellt, aber auch auf andere Weise verteilt oder vertrieben werden, beispielsweise über das Internet oder andere kabelgebundene oder kabellose Telekommunikationssysteme.

Die Bezugszeichen in den Ansprüchen sind nicht derart zu verstehen, dass der Gegenstand und der Schutzbereich der Ansprüche durch diese Bezugszeichen eingeschränkt ist.

Die Erfindung bezieht sich auf ein System, ein Verfahren und ein Computerprogramm zur Verringerung von Risiken medizinischer Eingriffe. Erfindungsgemäß werden Werte medizinischer Indikatoren eines prähabilitativen Zustand eines Patienten bereitgestellt, wobei darauf basierend eine Risikoklasse bestimmt wird, die indikativ für ein Risiko ist, das ein geplanter medizinischer Eingriff für den Patienten birgt. Basierend auf den Werten der medizinischen Indikatoren und/oder der Risikoklasse werden Parameter prähabilitativer Maßnahmen für den Patienten bestimmt, wobei die Maßnahmen geeignet sind, das Risiko, das der geplante medizinische Eingriff für den Patienten birgt, zu senken.

## Patentansprüche

1. System (100) zur Verringerung von Risiken medizinischer Eingriffe, wobei das System aufweist:
- eine Bereitstellungseinheit (101) zum Bereitstellen von Werten einer Mehrzahl medizinischer Indikatoren (10, 11, 12), die indikativ für einen prähabilitativen Zustand eines Patienten sind,
- eine Klassifizierungseinheit (102) zum Bestimmen einer Risikoklasse (20) für den Patienten basierend auf den Werten der Mehrzahl medizinischer Indikatoren (10, 11, 12), wobei die Risikoklasse indikativ für ein Risiko ist, das ein geplanter medizinischer Eingriff für den Patienten birgt, und
- eine Parametrisierungseinheit (103) zum Bestimmen von Werten einer Mehrzahl von Parametern (30) prähabilitativer Maßnahmen für den Patienten basierend auf den Werten der Mehrzahl medizinischer Indikatoren (10, 11, 12) und/oder der Risikoklasse (20), wobei die Maßnahmen geeignet sind, das Risiko, das der geplante medizinische Eingriff für den Patienten birgt, zu senken.

2. System (100) gemäß einem der vorhergehenden Ansprüche, wobei sich die prähabilitativen Maßnahmen auf ein körperliches Training des Patienten beziehen.

3. System (100) gemäß Anspruch 2, wobei das körperliche Training ein kardiovaskuläres Training ist und wobei die Parametrisierungseinheit (103) eingerichtet ist, basierend auf den Werten der Mehrzahl medizinischer Indikatoren (10) und/oder der Risikoklasse (20) Herzfrequenz-Schwellwerte als Parameter für das kardiovaskuläre Training zu bestimmen.

4. System (100) gemäß einem der vorhergehenden Ansprüche, ferner aufweisend eine Maßnahmenüberwachungseinheit (104), die eingerichtet ist, die prähabilitativen Maßnahmen zu überwachen durch a) Erfassen von für eine tatsächliche Durchführung der prähabilitativen Maßnahmen durch den Patienten indikativen Durchführungskennwerten (30, 31) und b) Bestimmen von für eine Durchführungsqualität indikativen Qualitätswerten (40) basierend auf den Durchführungskennwerten (30, 31) und vorbestimmten Schwellwerten für die Durchführungskennwerte (30, 31).

5. System (100) gemäß Anspruch 4, wobei die Maßnahmenüberwachungseinheit (104) ferner eingerichtet ist, eine Nutzereingabe (50) zu erfassen, die indikativ dafür ist, ob der Patient bei der Durchführung einer prähabilitativen Maßnahme medizinische Symptome erlebt hat, und basierend auf der Nutzereingabe (50) in Bezug auf die durchgeführte prähabilitative Maßnahme einen oder mehrere Symptomkennwerte zu bestimmen.

6. System (100) gemäß Anspruch 4, insbesondere gemäß Anspruch 5, wobei die Maßnahmenüberwachungseinheit (104) eingerichtet ist, basierend auf den bestimmten Qualitätswerten (40), insbesondere zusätzlich dem einen oder den mehreren bestimmten Symptomkennwerten, eine Ausgabeeinheit (131) des Systems dazu zu veranlassen, eine Ausgabe bereitzustellen, die geeignet ist, eine Maßnahmenüberprüfung durch den Arzt herbeizuführen.

7. System (100) gemäß einem der vorherigen Ansprüche, wobei die Bereitstellungseinheit (101) zum Bereitstellen erster Werte der Mehrzahl medizinischer Indikatoren (10) zu einem ersten Zeitpunkt und zum Bereitstellen zweiter Werte der Mehrzahl medizinischer Indikatoren (10) zu einem zweiten Zeitpunkt eingerichtet ist, wobei der erste Zeitpunkt einem Beginn und der zweite Zeitpunkt einem Ende eines für die prähabilitativen Maßnahmen vorgesehenen Maßnahmenzeitraums entsprechen, und wobei das System (100) ferner eine Wirkungsbestimmungseinheit (105) aufweist, die eingerichtet ist, basierend auf den ersten Werten und den zweiten Werten der Mehrzahl medizinischer Indikatoren (10) einen oder mehrere Wirkungskennwerte (60) zu bestimmen, der indikativ für eine Wirkung der prähabilitativen Maßnahmen auf das Risiko ist, das der geplante medizinische Eingriff für den Patienten birgt.

8. System (100) gemäß einem der vorherigen Ansprüche, wobei die Mehrzahl medizinischer Indikatoren (10) einen oder mehrere der folgenden umfasst:
• Geburtsjahr oder Alter,
• Größe,
• Gewicht,
• Body-Mass-Index,
• ob der Patient Raucher ist,
• Ruhepuls,
• ECOG-Status,
• TUG-Testergebnis,
• Hämoglobingehalt im Blut,
• ob der Patient herzfrequenzbeeinflussende, insbesondere herzfrequenzsenkende, Medikamente einnimmt, und
• RAI-Score, wobei die Bereitstellungseinheit (101) eingerichtet ist, den RAI-Score basierend auf einer Nutzereingabe zu bestimmen, die indikativ für die folgenden Umstände ist:
- ob der Patient Krebs hat,
- ob der Patient in den zurückliegenden drei Monaten einen unbeabsichtigten Gewichtsverlust erlitten hat,
- ob bei dem Patienten ein Nierenversagen vorliegt,
- ob bei dem Patienten eine Herzinsuffizienz vorliegt,
- ob der Patient unter Appetitlosigkeit leidet,
- ob der Patient unter Kurzatmigkeit, insbesondere in Ruhe, leidet,
- ob der Patient unabhängig wohnt, d. h. keine medizinische Alltagsversorgung in Anspruch nimmt, und falls nicht:
∘ ob der Patient a) in einer professionellen Pflegeeinrichtung, b) in betreutem Wohnen oder c) mit einem Pflegeheim lebt, und
∘ ob der Patient hierfür innerhalb der zurückliegenden drei Monate zugelassen wurde,
- eine Pflegeabhängigkeit des Patienten im Alltag hinsichtlich eines oder mehrerer der Folgenden: Mobilität, Nahrungsaufnahme, Toilettenbenutzung und persönliche Hygiene, und
- ob der kognitive Zustand des Patienten sich innerhalb der zurückliegenden drei Monate verschlechtert hat.

9. System (100) gemäß Anspruch 5 oder einer Kombination von Anspruch 5 mit einem der Ansprüche 6 bis 8, wobei die von der Nutzereingabe (50) indizierten medizinischen Symptome eines oder mehrere der folgenden umfassen:
• Herzstolpern,
• Brustschmerzen,
• Kurzatmigkeit,
• Müdigkeit,
• Übelkeit, und
• Muskelschmerzen.

10. System (100) gemäß einem der vorhergehenden Ansprüche, wobei die Mehrzahl medizinischer Indikatoren wenigstens einen (11) umfasst, die für eine subjektive Einschätzung des Patienten über seine Lebensqualität indikativ ist, und/oder wenigstens einen (12), der für ein Abschneiden des Patienten in einem körperlichen Belastungstest indikativ ist

11. System (100) gemäß einem der vorhergehenden Ansprüche, wobei die Bereitstellungseinheit (101) ferner eingerichtet ist, Werte einer Mehrzahl weiterer medizinischer Indikatoren (13) bereitzustellen, wobei die weiteren Indikatoren (13) indikativ für einen Zustand des Patienten nach dem medizinischen Eingriff sind.

12. System (100) gemäß Anspruch 11, wobei die Mehrzahl weiterer medizinischer Indikatoren (13), die indikativ für den Zustand des Patienten nach dem medizinischen Eingriff sind, indikativ für wenigstens eines der Folgenden ist:
• eine Diagnose,
• eine Therapie,
• eine Komplikation während des medizinischen Eingriffs, und
• ob der Patient noch lebt sowie,
- falls ja, Datum eines letzten Patientenkontakts,
- falls nein, den Todeszeitpunkt.

13. System (100) gemäß einer Kombination der Ansprüche 8 bis 12, wobei die bereitgestellten medizinischen Indikatoren (10, 11, 12, 13) alle der in den Ansprüchen 8, 10 und 12 genannten umfassen und die von der Maßnahmenüberwachungseinheit (104) erfasste Nutzereingabe (50) indikativ dafür ist, welches der in Anspruch 9 genannten medizinischen Symptome der Patient erlebt hat.

14. Verfahren (200) zur Verringerung von Risiken medizinischer Eingriffe, wobei das Verfahren (200) umfasst:
- Bereitstellen (201) von Werten einer Mehrzahl medizinischer Indikatoren (10, 11, 12), die indikativ für einen prähabilitativen Zustand eines Patienten sind,
- Bestimmen (202) einer Risikoklasse (20) für den Patienten basierend auf den empfangenen Werten der Mehrzahl medizinischer Indikatoren (10, 11, 12), wobei die Risikoklasse (20) indikativ für ein Risiko ist, das ein geplanter medizinischer Eingriff für den Patienten birgt, und
- Bestimmen (203) von Werten einer Mehrzahl von Parametern (30) prähabilitativer Maßnahmen für den Patienten basierend auf den Werten der Mehrzahl medizinischer Indikatoren (10, 11, 12) und/oder der Risikoklasse (20), wobei die Maßnahmen geeignet sind, das Risiko, das der geplante medizinische Eingriff für den Patienten birgt, zu senken.

15. Computerprogramm zur Verringerung von Risiken medizinischer Eingriffe, wobei das Programm Anweisungen aufweist, die geeignet sind, das System (100) gemäß einem der Ansprüche 1 bis 13 dazu zu veranlassen, das Verfahren (200) gemäß Anspruch 14 auszuführen.
